# EUROPEAN PATENT APPLICATION

(11) **EP 4 736 873 A1**
(43) Date of publication of application: **06.05.2026**
(21) Application number: 24830943.7
(22) Date of filing: 28.06.2024
(51) Int. Cl.: A61K 39/395, C07K 16/28, A61P 17/00

(54) **ANTI-IL-4RALPHA ANTIBODY FORMULATION, PREPARATION METHOD THEREFOR, AND USE THEREOF**

(30) Priority: 30.06.2023 CN 202310803412
(71) Applicant: Bio-Thera Solutions, Ltd., Guangzhou, Guangdong 510005 (CN)
(72) Inventor: LI, Yuanqiang, Guangzhou, Guangdong 510530 (CN); LIU, Cuihua, Guangzhou, Guangdong 510530 (CN); WU, Yong, Guangzhou, Guangdong 510530 (CN); LIU, Lulu, Guangzhou, Guangdong 510530 (CN); WU, Zhihao, Guangzhou, Guangdong 510530 (CN); MA, Zhimin, Guangzhou, Guangdong 510530 (CN)
(74) Representative: Engelhard, Markus
(86) International application number: PCT/CN2024/102258
(87) International publication number: WO 2025/002312

(57) **Abstract**

An anti-IL-4Rα antibody formulation, a preparation method thereof, and a use thereof. The antibody formulation comprises an anti-IL-4Ra antibody at 10-300 mg/mL, a buffer, a stabilizer, and a surfactant, and the pH of the antibody formulation is 5.0-7.0. The antibody formulation exhibits good stability under conditions of high temperature, room temperature, refrigeration, light, and the like, and can be kept in a liquid form, thus possessing convenience of use.

## Description

### TECHNICAL FIELD

The present disclosure relates to the field of biological formulations, and particularly, to an anti-IL-4Rα antibody formulation, preparation method thereof and use thereof.

### BACKGROUND

Interleukin-4 (IL-4, also known as B cell stimulating factor or BSF-1) was initially characterized by its ability to stimulate B cell proliferation in response to low concentrations of anti-surface immunoglobulin antibodies. IL-4 has been demonstrated to have a wide range of biological activities, including stimulating the growth of T cells, mast cells, granulocytes, megakaryocytes, and erythrocytes. IL-4 induces the expression of major histocompatibility complex class II molecules on resting B cells and enhances the secretion of IgE and IgG1 isotypes in stimulated B cells. The biological activity of IL-4 is mediated by specific cell surface IL-4 receptors.

Anti-IL-4R antibodies can be used to treat or prevent diseases such as atopic dermatitis, asthma, and other disorders. In order to utilize the anti-IL-4R antibody in the treatment of diseases, in-depth research is required on anti-IL-4R antibodies to develop a stable formulation suitable for clinical application.

### SUMMARY

The present disclosure provides a stable antibody formulation comprising an antibody capable of specifically binding to human interleukin-4 receptor α (hIL-4Rα).

In some embodiments, the antibody formulation of the present disclosure comprises an anti-IL-4Rα antibody at 10-300 mg/mL or 10-200 mg/mL, a buffer, a stabilizer, and a surfactant.

In some embodiments, the anti-IL-4Rα antibody comprises the heavy chain variable region set forth in SEQ ID NO: 1 and the light chain variable region set forth in SEQ ID NO: 2.

In some embodiments, the heavy chain of the anti-IL-4Rα antibody comprises the sequence set forth in SEQ ID NO: 3, or a sequence having at least 90% identity to the sequence set forth in SEQ ID NO: 3, or an amino acid sequence having one or more conservative amino acid substitutions as compared to the sequence set forth in SEQ ID NO: 3; and/or
the light chain of the anti-IL-4Rα antibody comprises the sequence set forth in SEQ ID NO: 4, or a sequence having at least 90% identity to the sequence set forth in SEQ ID NO: 4, or an amino acid sequence having one or more conservative amino acid substitutions as compared to the sequence set forth in SEQ ID NO: 4.

In some embodiments, the heavy chain of the anti-IL-4Rα antibody comprises the sequence set forth in SEQ ID NO: 3, and the light chain of the anti-IL-4Rα antibody comprises the sequence set forth in SEQ ID NO: 4.

In some embodiments, the anti-IL-4Rα antibody is dupilumab (e.g., Dupixent^{®} or biosimilars thereof).

In some embodiments, the concentration of the anti-IL-4Rα antibody is 20-250 mg/mL. In some embodiments, the concentration of the anti-IL-4Rα antibody is 20-200 mg/mL. In some embodiments, the concentration of the anti-IL-4Rα antibody is 40-200 mg/mL. In some embodiments, the concentration of the anti-IL-4Rα antibody is 20-150 mg/mL. In some embodiments, the concentration of the anti-IL-4Rα antibody is 100-200 mg/mL. In some embodiments, the concentration of the anti-IL-4Ra antibody is 120-180 mg/mL. In some embodiments, the concentration of the anti-IL-4Rα antibody is 145-180 mg/mL. In some embodiments, the concentration of the anti-IL-4Rα antibody is 20-80 mg/mL. In some embodiments, the concentration of the anti-IL-4Rα antibody in the antibody formulation is about 20 mg/mL, about 30 mg/mL, about 40 mg/mL, about 47 mg/mL, about 50 mg/mL, about 60 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 175 mg/mL, about 180 mg/mL, about 190 mg/mL, about 200 mg/mL, about 210 mg/mL, about 220 mg/mL, about 230 mg/mL, about 240 mg/mL, about 250 mg/mL, about 300 mg/mL, or a range between any two of these values (inclusive) or any value therein. In some embodiments, the concentration of the anti-IL-4Rα antibody in the antibody formulation is about 150 mg/mL. In some embodiments, the concentration of the anti-IL-4Rα antibody in the antibody formulation is about 175 mg/mL.

In some embodiments, the stabilizer is selected from one or more of sucrose, trehalose, mannitol, arginine or a pharmaceutically acceptable salt thereof, glycine or a pharmaceutically acceptable salt thereof, proline or a pharmaceutically acceptable salt thereof, and methionine or a pharmaceutically acceptable salt thereof. In some embodiments, the stabilizer is selected from proline, methionine, glycine, and a combination thereof. In some embodiments, the stabilizer comprises glycine, proline, or a combination of proline and methionine, or a pharmaceutically acceptable salt thereof. In some embodiments, the stabilizer comprises proline or a pharmaceutically acceptable salt thereof. In some embodiments, the stabilizer comprises proline or a pharmaceutically acceptable salt thereof and methionine or a pharmaceutically acceptable salt thereof.

In some embodiments, the concentration of the stabilizer is 50-500 mM. In some embodiments, the concentration of the stabilizer is 100-450 mM. In some embodiments, the concentration of the stabilizer is 120-350 mM. In some embodiments, the concentration of the stabilizer is 145-330 mM. In some embodiments, the concentration of the stabilizer is about 50 mM, about 60 mM, about 70 mM, about 80 mM, about 90 mM, about 100 mM, about 110 mM, about 120 mM, about 130 mM, about 146 mM, about 150 mM, about 154 mM, about 160 mM, about 164 mM, about 170 mM, about 180 mM, about 190 mM, about 201 mM, about 217 mM, about 220 mM, about 227 mM, about 230 mM, about 234 mM, about 237 mM, about 240 mM, about 247 mM, about 250 mM, about 260 mM, about 270 mM, about 280 mM, about 290 mM, about 293 mM, about 300 mM, about 320 mM, about 325 mM, about 350 mM, about 380 mM, about 400 mM, about 450 mM, about 500 mM, or a range between any two of these values (inclusive) or any value therein.

In some embodiments, the stabilizer comprises proline or a pharmaceutically acceptable salt thereof at 100-300 mM. In some embodiments, the stabilizer comprises proline or a pharmaceutically acceptable salt thereof at 180-230 mM. In some embodiments, the stabilizer comprises proline or a pharmaceutically acceptable salt thereof at about 217 mM. In some embodiments, the stabilizer comprises proline or a pharmaceutically acceptable salt thereof at about 191 mM.

In some embodiments, the stabilizer comprises proline or a pharmaceutically acceptable salt thereof at 100-300 mM and methionine or a pharmaceutically acceptable salt thereof at 1-30 mM. In some embodiments, the stabilizer comprises proline or a pharmaceutically acceptable salt thereof at 180-230 mM and methionine or a pharmaceutically acceptable salt thereof at 5-15 mM. In some embodiments, the stabilizer comprises proline or a pharmaceutically acceptable salt thereof at about 217 mM and methionine or a pharmaceutically acceptable salt thereof at about 10 mM. In some embodiments, the stabilizer comprises proline or a pharmaceutically acceptable salt thereof at about 191 mM and methionine or a pharmaceutically acceptable salt thereof at about 10 mM.

In some embodiments, the stabilizer comprises sucrose at 20-100 mM and arginine or a pharmaceutically acceptable salt thereof at 101-300 mM. In some embodiments, the stabilizer comprises sucrose at 35-90 mM and arginine or a pharmaceutically acceptable salt thereof at 101-245 mM. In some embodiments, the pharmaceutically acceptable salt of arginine is arginine hydrochloride or arginine acetate. In some embodiments, the stabilizer comprises sucrose at about 44 mM and arginine hydrochloride at about 110 mM. In some embodiments, the stabilizer comprises sucrose at about 88 mM and arginine hydrochloride at about 237 mM. In some embodiments, the stabilizer further comprises methionine at 1-30 mM. In some embodiments, the stabilizer comprises sucrose at 20-100 mM, arginine or a pharmaceutically acceptable salt thereof at 101-300 mM, and methionine at 1-30 mM. In some embodiments, the concentration of methionine is about 10 mM. In some embodiments, the stabilizer comprises sucrose at about 44 mM, arginine hydrochloride at about 110 mM, and methionine at about 10 mM. In some embodiments, the stabilizer further comprises EDTA at 0.01-0.5 mM. In some embodiments, the concentration of EDTA is about 0.05 mM.

In some embodiments, the pH of the antibody formulation is 5.0-7.0. In some embodiments, the pH of the antibody formulation is 5.3-6.5. In some embodiments, the pH of the antibody formulation is 5.9 ± 0.3. In some embodiments, the pH of the antibody formulation is about 5.0, about 5.3, about 5.6, about 5.7, about 5.8, about 5.9, about 6.0, about 6.1, about 6.2, about 6.3, about 6.4, about 6.5, about 6.8, about 7.0, or a range between any two of these values (inclusive) or any value therein.

In some embodiments, the buffer is selected from histidine, citric acid, acetic acid, histidine-acetic acid, and histidine-citric acid. In some embodiments, the buffer is histidine-acetic acid. In some embodiments, the concentration of the buffer is 1-80 mM. In some embodiments, the concentration of the buffer is 5-80 mM. In some embodiments, the concentration of the buffer is 10-80 mM. In some embodiments, the concentration of the buffer is 10-60 mM. In some embodiments, the concentration of the buffer is 15-60 mM. In some embodiments, the concentration of the buffer is 20-55 mM. In some embodiments, the concentration of the buffer is 20-40 mM. In some embodiments, the concentration of the buffer is 35-55 mM. In some embodiments, the concentration of the buffer is about 1 mM, about 3 mM, about 5 mM, about 8 mM, about 10 mM, about 12 mM, about 15 mM, about 16 mM, about 18 mM, about 19 mM, about 20 mM, about 21 mM, about 22 mM, about 23 mM, about 25 mM, about 27 mM, about 30 mM, about 35 mM, about 40 mM, about 50 mM, about 60 mM, about 70 mM, about 80 mM, or a range between any two of these values (inclusive) or any value therein. In some embodiments, the buffer is a histidine buffer at about 20 mM. In some embodiments, the histidine buffer comprises histidine and histidine hydrochloride. In some embodiments, the buffer comprises histidine at about 20 mM and acetic acid at 26 ± 15 mM. In some embodiments, the buffer comprises histidine at about 20 mM and acetic acid at 26 ± 5 mM.

In some embodiments, the surfactant is polysorbate or poloxamer. In some embodiments, the surfactant is polysorbate 80, polysorbate 20, or poloxamer 188. In some embodiments, the surfactant is polysorbate 80. In some embodiments, the concentration of the surfactant is 0.1-5 mg/mL. In some embodiments, the concentration of the surfactant is 0.5-5 mg/mL. In some embodiments, the concentration of the surfactant is 0.5-3 mg/mL. In some embodiments, the concentration of the surfactant is about 0.1 mg/mL, 0.2 mg/mL, 0.3 mg/mL, 0.5 mg/mL, 1 mg/mL, 2 mg/mL, 3 mg/mL, 5 mg/mL, or a range between any two of these values (inclusive) or any value therein. In some embodiments, the surfactant is polysorbate 80 at 0.5-3 mg/mL. In some embodiments, the surfactant is polysorbate 80 at 1-2 mg/mL. In some embodiments, the surfactant is polysorbate 80 at about 2 mg/mL.

In some embodiments, the antibody formulation is free of or substantially free of an acetate.

In some embodiments, the antibody formulation is free of arginine or a pharmaceutically acceptable salt thereof.

In some embodiments, the antibody formulation is free of arginine or a pharmaceutically acceptable salt thereof and sucrose.

In some embodiments, the antibody formulation comprises an anti-IL-4Ra antibody at 20-250 mg/mL, a buffer at 5-80 mM, a stabilizer at 100-450 mM, and a surfactant. In some embodiments, the antibody formulation comprises an anti-IL-4Rα antibody at 20-200 mg/mL, a buffer at 5-80 mM, a stabilizer at 200-450 mM, and a surfactant. In some embodiments, the antibody formulation comprises an anti-IL-4Rα antibody at 20-200 mg/mL, a buffer at 5-80 mM, a stabilizer at 210-450 mM, and a surfactant.

In some embodiments, the present disclosure provides an antibody formulation comprising an anti-IL-4Rα antibody. The antibody formulation comprises an anti-IL-4Rα antibody at 20-250 mg/mL, a buffer at 5-80 mM, a stabilizer at 100-450 mM, and polysorbate 80 or poloxamer 188 at 0.1-5 mg/mL, and the pH of the antibody formulation is 5.3-6.5. In some embodiments, the present disclosure provides an antibody formulation comprising an anti-IL-4Rα antibody. The antibody formulation comprises an anti-IL-4Rα antibody at 20-200 mg/mL, a buffer at 5-80 mM, a stabilizer at 100-450 mM, and polysorbate 80 or poloxamer 188 at 0.1-5 mg/mL, and the pH of the antibody formulation is 5.3-6.5.

In some embodiments, the antibody formulation comprises an anti-IL-4Rα antibody at 20-100 mg/mL, a buffer at 10-30 mM, a stabilizer at 120-350 mM, and polysorbate 80 or poloxamer 188 at 0.5-3 mg/mL, and the pH of the antibody formulation is 5.3-6.5.

In some embodiments, the antibody formulation comprises an anti-IL-4Rα antibody at 45-55 mg/mL, a buffer at 10-30 mM, a stabilizer at 145-330 mM, and polysorbate 80 or poloxamer 188 at 1-3 mg/mL, and the pH of the antibody formulation is 5.3-6.5.

In some embodiments, the antibody formulation comprises an anti-IL-4Rα antibody at 100-250 mg/mL, a buffer at 5-80 mM, a stabilizer at 120-350 mM, and polysorbate 80 or poloxamer 188 at 0.1-5 mg/mL, and the pH of the antibody formulation is 5.3-6.5.

In some embodiments, the antibody formulation comprises an anti-IL-4Rα antibody at 100-200 mg/mL, a buffer at 5-80 mM, a stabilizer at 120-350 mM, and polysorbate 80 or poloxamer 188 at 0.5-3 mg/mL, and the pH of the antibody formulation is 5.3-6.5.

In some embodiments, the antibody formulation comprises an anti-IL-4Rα antibody at 130-200 mg/mL, a buffer at 10-60 mM, a stabilizer at 145-330 mM, and polysorbate 80 or poloxamer 188 at 0.5-3 mg/mL, and the pH of the antibody formulation is 5.3-6.5.

In some embodiments, the buffer is selected from histidine, citric acid, acetic acid, histidine-acetic acid, and histidine-citric acid. In some embodiments, the buffer is histidine-acetic acid. In some embodiments, the stabilizer is selected from glycine or a pharmaceutically acceptable salt thereof, proline or a pharmaceutically acceptable salt thereof, methionine or a pharmaceutically acceptable salt thereof, and a combination thereof. In some embodiments, the stabilizer is selected from glycine or a pharmaceutically acceptable salt thereof, proline or a pharmaceutically acceptable salt thereof, and a combination of proline or a pharmaceutically acceptable salt thereof and methionine or a pharmaceutically acceptable salt thereof. In some embodiments, the stabilizer is selected from glycine, proline, and a combination of proline and methionine. In some embodiments, the anti-IL-4Rα antibody comprises the heavy chain variable region set forth in SEQ ID NO: 1 and the light chain variable region set forth in SEQ ID NO: 2.

In some embodiments, the antibody formulation comprises an anti-IL-4Rα antibody at 20-250 mg/mL, a buffer, proline, and polysorbate or poloxamer. In some embodiments, the antibody formulation comprises an anti-IL-4Rα antibody at 20-200 mg/mL, a buffer, proline, and polysorbate or poloxamer.

In some embodiments, the antibody formulation comprises an anti-IL-4Rα antibody at 20-250 mg/mL, a buffer at 5-80 mM, proline, and polysorbate 80 or poloxamer 188 at 0.1-5 mg/mL, and the pH of the antibody formulation is 5.3-6.5. In some embodiments, the antibody formulation comprises an anti-IL-4Rα antibody at 20-200 mg/mL, a buffer at 5-80 mM, proline, and polysorbate 80 or poloxamer 188 at 0.1-5 mg/mL, and the pH of the antibody formulation is 5.3-6.5.

In some embodiments, the antibody formulation comprises an anti-IL-4Rα antibody at 20-250 mg/mL, a buffer at 5-80 mM, proline at 100-300 mM, and polysorbate 80 or poloxamer 188 at 0.1-5 mg/mL, and the pH of the antibody formulation is 5.3-6.5. In some embodiments, the antibody formulation comprises an anti-IL-4Rα antibody at 20-200 mg/mL, a buffer at 5-80 mM, proline at 100-300 mM, and polysorbate 80 or poloxamer 188 at 0.1-5 mg/mL, and the pH of the antibody formulation is 5.3-6.5.

In some embodiments, the antibody formulation comprises an anti-IL-4Rα antibody at 20-250 mg/mL, a buffer at 5-80 mM, proline at 100-300 mM, and polysorbate 80 or poloxamer 188 at 0.5-3 mg/mL, and the pH of the antibody formulation is 5.3-6.5. In some embodiments, the antibody formulation comprises an anti-IL-4Rα antibody at 20-200 mg/mL, a buffer at 5-80 mM, proline at 100-300 mM, and polysorbate 80 or poloxamer 188 at 0.5-3 mg/mL, and the pH of the antibody formulation is 5.3-6.5.

In some embodiments, the antibody formulation comprises an anti-IL-4Rα antibody at 45-55 mg/mL, a buffer at 10-30 mM, proline at 150-250 mM, and polysorbate 80 or poloxamer 188 at 1-3 mg/mL, and the pH of the antibody formulation is 5.3-6.5.

In some embodiments, the antibody formulation comprises an anti-IL-4Rα antibody at 100-250 mg/mL, a buffer at 5-80 mM, proline at 150-250 mM, and polysorbate 80 or poloxamer 188 at 0.5-3 mg/mL, and the pH of the antibody formulation is 5.3-6.5. In some embodiments, the antibody formulation comprises an anti-IL-4Rα antibody at 100-200 mg/mL, a buffer at 5-80 mM, proline at 150-250 mM, and polysorbate 80 or poloxamer 188 at 0.5-3 mg/mL, and the pH of the antibody formulation is 5.3-6.5.

In some embodiments, the antibody formulation comprises an anti-IL-4Rα antibody at 20-250 mg/mL, a buffer, proline, methionine, and polysorbate or poloxamer. In some embodiments, the antibody formulation comprises an anti-IL-4Rα antibody at 20-200 mg/mL, a buffer, proline, methionine, and polysorbate or poloxamer.

In some embodiments, the antibody formulation comprises an anti-IL-4Rα antibody at 20-250 mg/mL, a buffer at 5-80 mM, proline, methionine, and polysorbate 80 or poloxamer 188 at 0.1-5 mg/mL, and the pH of the antibody formulation is 5.3-6.5. In some embodiments, the antibody formulation comprises an anti-IL-4Rα antibody at 20-200 mg/mL, a buffer at 5-80 mM, proline, methionine, and polysorbate 80 or poloxamer 188 at 0.1-5 mg/mL, and the pH of the antibody formulation is 5.3-6.5.

In some embodiments, the antibody formulation comprises an anti-IL-4Rα antibody at 20-250 mg/mL, a buffer at 5-80 mM, proline at 100-300 mM, methionine at 5-30 mM, and polysorbate 80 or poloxamer 188 at 0.1-5 mg/mL, and the pH of the antibody formulation is 5.3-6.5. In some embodiments, the antibody formulation comprises an anti-IL-4Rα antibody at 20-200 mg/mL, a buffer at 5-80 mM, proline at 100-300 mM, methionine at 5-30 mM, and polysorbate 80 or poloxamer 188 at 0.1-5 mg/mL, and the pH of the antibody formulation is 5.3-6.5.

In some embodiments, the antibody formulation comprises an anti-IL-4Rα antibody at 20-250 mg/mL, a buffer at 5-80 mM, proline at 100-300 mM, methionine at 5-30 mM, and polysorbate 80 or poloxamer 188 at 0.5-3 mg/mL, and the pH of the antibody formulation is 5.3-6.5. In some embodiments, the antibody formulation comprises an anti-IL-4Rα antibody at 20-200 mg/mL, a buffer at 5-80 mM, proline at 100-300 mM, methionine at 5-30 mM, and polysorbate 80 or poloxamer 188 at 0.5-3 mg/mL, and the pH of the antibody formulation is 5.3-6.5.

In some embodiments, the antibody formulation comprises an anti-IL-4Rα antibody at 45-55 mg/mL, a buffer at 10-30 mM, proline at 150-250 mM, methionine at 5-30 mM, and polysorbate 80 or poloxamer 188 at 1-3 mg/mL, and the pH of the antibody formulation is 5.3-6.5.

In some embodiments, the antibody formulation comprises an anti-IL-4Rα antibody at 100-250 mg/mL, a buffer at 5-80 mM, proline at 150-250 mM, methionine at 5-30 mM, and polysorbate 80 or poloxamer 188 at 0.5-3 mg/mL, and the pH of the antibody formulation is 5.3-6.5. In some embodiments, the antibody formulation comprises an anti-IL-4Rα antibody at 100-200 mg/mL, a buffer at 5-80 mM, proline at 150-250 mM, methionine at 5-30 mM, and polysorbate 80 or poloxamer 188 at 0.5-3 mg/mL, and the pH of the antibody formulation is 5.3-6.5.

In some embodiments, the antibody formulation comprises an anti-IL-4Rα antibody at 20-250 mg/mL, a buffer at 5-80 mM, sucrose at 20-100 mM, arginine or a pharmaceutically acceptable salt thereof at 101-300 mM, and polysorbate 80 or poloxamer 188 at 0.1-5 mg/mL, and the pH of the antibody formulation is 5.3-6.5. In some embodiments, the antibody formulation comprises an anti-IL-4Rα antibody at 20-200 mg/mL, a buffer at 5-80 mM, sucrose at 20-100 mM, arginine or a pharmaceutically acceptable salt thereof at 101-300 mM, and polysorbate 80 or poloxamer 188 at 0.1-5 mg/mL, and the pH of the antibody formulation is 5.3-6.5.

In some embodiments, the antibody formulation comprises an anti-IL-4Rα antibody at 20-250 mg/mL, a buffer at 5-80 mM, sucrose at 20-100 mM, arginine hydrochloride at 101-300 mM, and polysorbate 80 or poloxamer 188 at 0.5-3 mg/mL, and the pH of the antibody formulation is 5.3-6.5. In some embodiments, the antibody formulation comprises an anti-IL-4Rα antibody at 20-200 mg/mL, a buffer at 5-80 mM, sucrose at 20-100 mM, arginine hydrochloride at 101-300 mM, and polysorbate 80 or poloxamer 188 at 0.5-3 mg/mL, and the pH of the antibody formulation is 5.3-6.5.

In some embodiments, the antibody formulation comprises an anti-IL-4Rα antibody at 45-55 mg/mL, a buffer at 10-30 mM, sucrose at 35-90 mM, arginine hydrochloride at 101-245 mM, and polysorbate 80 or poloxamer 188 at 1-3 mg/mL, and the pH of the antibody formulation is 5.3-6.5. In some embodiments, the antibody formulation comprises an anti-IL-4Rα antibody at 100-250 mg/mL, a buffer at 5-80 mM, sucrose at 35-90 mM, arginine hydrochloride at 101-245 mM, and polysorbate 80 or poloxamer 188 at 0.5-3 mg/mL, and the pH of the antibody formulation is 5.3-6.5. In some embodiments, the antibody formulation comprises an anti-IL-4Rα antibody at 100-200 mg/mL, a buffer at 5-80 mM, sucrose at 35-90 mM, arginine hydrochloride at 101-245 mM, and polysorbate 80 or poloxamer 188 at 0.5-3 mg/mL, and the pH of the antibody formulation is 5.3-6.5.

In some embodiments, the buffer is selected from histidine, citric acid, acetic acid, histidine-acetic acid, and histidine-citric acid. In some embodiments, the buffer is selected from histidine-acetic acid. In some embodiments, the anti-IL-4Rα antibody comprises the heavy chain variable region set forth in SEQ ID NO: 1 and the light chain variable region set forth in SEQ ID NO: 2.

In some embodiments, the antibody formulation comprises an anti-IL-4Rα antibody at about 50 mg/mL, histidine at about 20 mM, sucrose at about 146 mM (equivalent to sucrose at about 5%), and polysorbate 80 at about 2 mg/mL (equivalent to polysorbate 80 at about 0.2%), and the pH of the antibody formulation is 5.3-6.5.

In some embodiments, the antibody formulation comprises an anti-IL-4Rα antibody at about 50 mg/mL, citric acid at about 20 mM, sucrose at about 146 mM, and polysorbate 80 at about 2 mg/mL, and the pH of the antibody formulation is 5.3-6.5.

In some embodiments, the antibody formulation comprises an anti-IL-4Rα antibody at about 50 mg/mL, histidine at about 20 mM, sucrose at about 234 mM (equivalent to sucrose at about 8%), and polysorbate 80 at about 2 mg/mL, and the pH of the antibody formulation is 5.9 ± 0.3.

In some embodiments, the antibody formulation comprises an anti-IL-4Rα antibody at about 50 mg/mL, histidine at about 20 mM, trehalose at about 225 mM (equivalent to trehalose dihydrate at about 8.5%), and polysorbate 80 at about 2 mg/mL, and the pH of the antibody formulation is 5.9 ± 0.3.

In some embodiments, the antibody formulation comprises an anti-IL-4Rα antibody at about 50 mg/mL, histidine at about 20 mM, glycine at about 293 mM (equivalent to glycine at about 2.2%), and polysorbate 80 at about 2 mg/mL, and the pH of the antibody formulation is 5.9 ± 0.3.

In some embodiments, the antibody formulation comprises an anti-IL-4Rα antibody at about 50 mg/mL, histidine at about 20 mM, mannitol at about 247 mM (equivalent to mannitol at about 4.5%), and polysorbate 80 at about 2 mg/mL, and the pH of the antibody formulation is 5.9 ± 0.3. In some embodiments, the antibody formulation comprises an anti-IL-4Rα antibody at about 50 mg/mL, histidine at about 20 mM, arginine hydrochloride at about 237 mM (equivalent to arginine hydrochloride at about 5%), and polysorbate 80 at about 2 mg/mL, and the pH of the antibody formulation is 5.9 ± 0.3.

In some embodiments, the antibody formulation comprises an anti-IL-4Rα antibody at about 50 mg/mL, histidine at about 20 mM, sucrose at about 88 mM (equivalent to sucrose at about 3%), arginine hydrochloride at about 237 mM, and polysorbate 80 at about 2 mg/mL, and the pH of the antibody formulation is 5.9 ± 0.3.

In some embodiments, the antibody formulation comprises an anti-IL-4Rα antibody at about 50 mg/mL, histidine at about 20 mM, proline at about 217 mM (equivalent to proline at about 2.5%), and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 20 mM, arginine hydrochloride at about 110 mM, sucrose at about 44 mM (equivalent to sucrose at about 1.5%), and polysorbate 80 at about 1 mg/mL (equivalent to polysorbate 80 at about 0.1%), and the pH of the antibody formulation is 5.9 ± 0.3.

In some embodiments, the antibody formulation comprises an anti-IL-4Rα antibody at about 175 mg/mL, histidine at about 20 mM, arginine hydrochloride at about 110 mM, sucrose at about 44 mM, EDTA at about 0.05 mM, and polysorbate 80 at about 1 mg/mL, and the pH of the antibody formulation is 5.9 ± 0.3.

In some embodiments, the antibody formulation comprises an anti-IL-4Rα antibody at about 175 mg/mL, histidine at about 20 mM, arginine hydrochloride at about 110 mM, sucrose at about 44 mM, methionine at about 10 mM, and polysorbate 80 at about 1 mg/mL, and the pH of the antibody formulation is 5.9 ± 0.3.

In some embodiments, the antibody formulation comprises an anti-IL-4Rα antibody at about 175 mg/mL, histidine at about 20 mM, citric acid at about 20 mM, arginine hydrochloride at about 110 mM, sucrose at about 44 mM, and polysorbate 80 at about 1 mg/mL, and the pH of the antibody formulation is 5.9 ± 0.3.

In some embodiments, the antibody formulation comprises an anti-IL-4Rα antibody at about 175 mg/mL, histidine at about 20 mM, acetic acid at 33 ± 5 mM, proline at about 217 mM, methionine at about 10 mM, and polysorbate 80 at about 1 mg/mL, and the pH of the antibody formulation is 5.9 ± 0.3.

In some embodiments, the antibody formulation comprises an anti-IL-4Rα antibody at about 175 mg/mL, histidine at about 20 mM, arginine hydrochloride at about 110 mM, sucrose at about 44 mM, and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 20 mM, arginine hydrochloride at about 110 mM, sucrose at about 44 mM, and poloxamer 188 at about 0.5 mg/mL (equivalent to poloxamer 188 at about 0.05%), and the pH of the antibody formulation is 5.9 ± 0.3.

In some embodiments, the antibody formulation comprises an anti-IL-4Rα antibody at about 175 mg/mL, histidine at about 20 mM, proline at about 217 mM, and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 10 mM, proline at about 217 mM, and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 20 mM, acetic acid at 31 ± 5 mM, proline at about 217 mM, and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 20 mM, acetic acid at 26 ± 5 mM, proline at about 217 mM, and polysorbate 80 at about 2 mg/mL, and the pH of the antibody formulation is 5.9 ± 0.3.

In some embodiments, the antibody formulation comprises an anti-IL-4Rα antibody at about 150 mg/mL, histidine at about 20 mM, acetic acid at 26 ± 5 mM, proline at about 217 mM, and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 10 mM, acetic acid at 20 ± 5 mM, proline at about 191 mM, and polysorbate 80 at about 2 mg/mL, and the pH of the antibody formulation is 5.9 ± 0.3.

In some embodiments, the antibody formulation comprises an anti-IL-4Rα antibody at about 150 mg/mL, histidine at about 10 mM, acetic acid at 20 ± 5 mM, proline at about 191 mM, and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 20 mM, proline at about 217 mM, methionine at about 10 mM, and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 10 mM, proline at about 217 mM, methionine at about 10 mM, and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 20 mM, acetic acid at 31 ± 5 mM, proline at about 217 mM, methionine at about 10 mM, and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 20 mM, acetic acid at 26 ± 5 mM, proline at about 217 mM, methionine at about 10 mM, and polysorbate 80 at about 2 mg/mL, and the pH of the antibody formulation is 5.9 ± 0.3.

In some embodiments, the antibody formulation comprises an anti-IL-4Rα antibody at about 150 mg/mL, histidine at about 20 mM, acetic acid at 26 ± 5 mM, proline at about 217 mM, methionine at about 10 mM, and polysorbate 80 at about 2 mg/mL, and the pH of the antibody formulation is 5.9 ± 0.3.

In some embodiments, the antibody formulation comprises an anti-IL-4Rα antibody at about 175 mg/mL, histidine at about 10 mM, acetic acid at 20 ± 5 mM, proline at about 191 mM, methionine at about 10 mM, and polysorbate 80 at about 2 mg/mL, and the pH of the antibody formulation is 5.9 ± 0.3.

In some embodiments, the antibody formulation comprises an anti-IL-4Rα antibody at about 150 mg/mL, histidine at about 10 mM, acetic acid at 20 ± 5 mM, proline at about 191 mM, methionine at about 10 mM, and polysorbate 80 at about 2 mg/mL, and the pH of the antibody formulation is 5.9 ± 0.3.

In some embodiments, the antibody formulation is a liquid formulation.

In some embodiments, the liquid formulation comprises water.

In some embodiments, the present disclosure provides a method for preparing the antibody formulation, comprising: taking components according to the formula, dissolving in water, mixing the mixture thoroughly, and adjusting the pH value to 5.0-7.0 to obtain the antibody formulation. In some embodiments, the present disclosure provides a method for preparing the antibody formulation, comprising: preparing a buffer, transferring the anti-IL-4Rα antibody into the buffer by ultrafiltration, adding an excipient, and diluting the antibody to a specified concentration to obtain the antibody formulation.

In some embodiments, the antibody formulation is a lyophilized formulation obtained by lyophilizing the antibody formulation described herein.

In some embodiments, the antibody formulation is a reconstituted formulation obtained by reconstituting the lyophilized formulation described herein.

In some embodiments, the antibody formulation is suitable for intravenous or subcutaneous injection.

In some embodiments, the present disclosure provides use of the antibody formulation described herein in preparing a medicament for treating a disease. In some embodiments, the disease is a disease associated with IL-4R overexpression. In some embodiments, the disease is an immune disease or disorder. In some embodiments, the disease is selected from asthma, nasal polyp, eczema, sinusitis, chronic sinusitis, chronic sinusitis with nasal polyp, allergic dermatosis, chronic obstructive pulmonary disease, allergic rhinitis, arthritis, inflammatory disease, allergic reaction, autoimmune lymphoproliferative syndrome, autoimmune hemolytic anemia, Barrett's esophagus, autoimmune uveitis, tuberculosis, nephropathy, prurigo, prurigo nodularis, eosinophilic esophagitis, atopic dermatitis, urticaria, chronic urticaria, pruritus, bullous pemphigoid, allergic bronchopulmonary aspergillosis, neurodermitis, ulcerative colitis, milk anaphylaxis, and peanut allergy.

In some embodiments, the present disclosure provides an article of manufacture, comprising the antibody formulation described herein and a container for storing the antibody formulation. In some embodiments, the article of manufacture further comprises instructions for use. The container may be any container conventionally used in the art for storing drugs, such as pre-filled containers (e.g., pre-filled syringes), injection pens, bottles (e.g., vials), centrifuge tubes, sachets, and the like. The antibody formulation of the present disclosure exhibits good stability under conditions of high temperature, room temperature, refrigeration, light, and the like, and can be kept in a liquid form, thus possessing convenience of use.

In some embodiments, the antibody formulation of the present disclosure is stable after being stored at a high temperature (40 ± 2 °C) for at least 2 weeks. In some embodiments, the antibody formulation of the present disclosure is stable after being stored at a high temperature (40 ± 2 °C) for at least 4 weeks. In some embodiments, after the antibody formulation of the present disclosure is stored under light (4500 ± 500 lx, 25 ± 2 °C) for 14 days, the SEC-HPLC monomer and CE-SDS (NR) main peak are still 92% or higher. In some embodiments, after Antibody Formulations 5-3 and 5-4 are stored in accelerated conditions (away from light, 25 ± 2 °C) for 6 months, the SEC-HPLC monomers are still 98% or higher. In some embodiments, after Antibody Formulations 5-3 and 5-4 are stored in accelerated conditions (away from light, 25 ± 2 °C) for 6 months, the CE-SDS (NR) main peaks are still 96% or higher. In some embodiments, after Antibody Formulations 5-3 and 5-4 are stored at 2-8 °C (away from light) for 12 months, the SEC-HPLC monomers are still 98% or higher, and the SEC-HPLC, IEC-HPLC, and CE-SDS(NR) purities have no significant changes, indicating good long-term stability.

### DETAILED DESCRIPTION

The present disclosure will be illustrated, rather than limited, by the following specific embodiments.

It should be noted that, in the present disclosure, the "%" related to a formulation component refers to a weight/volume (w/v) percentage. For example, a stabilizer at 1% in a formulation means that 1 g of the stabilizer is contained in 100 mL of the formulation, or the content of the stabilizer is 0.01 g/mL.

The "about" or "approximately" refers to a general error range for corresponding values as readily understood by those skilled in the relevant art. In some embodiments, the "about" or "approximately" mentioned herein refers to the numerical values described as well as their ranges of ± 10%, ± 5%, or ± 1%.

The "comprise" or "include" means that the compositions, methods, and the like comprise the listed elements (e.g., components of the compositions, steps of the methods, and the like), but do not exclude the others. When used to define the compositions and methods, the "consisting essentially of..." means excluding other elements that have a fundamental impact on the combination for its intended use, but not excluding elements that do not substantially affect the characteristics of the compositions or methods. The "consisting of..." means excluding elements not specifically listed. Embodiments defined by each of these transition terms are all within the scope of the present disclosure. For example, when a composition is described as including components A, B, and C, a composition consisting essentially of A, B, and C, and a composition consisting of A, B, and C are independently within the scope of the present disclosure.

The term "buffer", also known as a buffer system, includes, but is not limited to, an organic acid and a salt thereof, such as succinic acid, acetic acid, citric acid, ascorbic acid, gluconic acid, carbonic acid, tartaric acid, or phthalic acid, and a salt thereof; Tris, or an inorganic acid and a salt thereof, such as a phosphate buffer. In addition, amino acids may also be used as the buffer. Such amino acids include, but are not limited to, glycine, histidine, arginine, lysine, omithine, isoleucine, leucine, alanine, glutamic acid, or aspartic acid, and a salt thereof.

The amount of the buffer in the present disclosure means the total amount of the buffer pair in the buffer system constituting the buffer. In some embodiments, the molar concentration is taken as the unit of the amount of the buffer, the value of which refers to the molar concentration of the buffer pair in the buffer system of the buffer. For example, where a histidine buffer consisting of histidine and histidine hydrochloride is used, a given concentration of the histidine buffer (e.g., 20 mM) is the combined concentration of histidine and histidine hydrochloride.

The formulation of the present disclosure can be prepared with the excipient or a hydrate thereof. For example, the histidine hydrochloride may be anhydrous histidine hydrochloride, or histidine hydrochloride hydrate, such as histidine hydrochloride monohydrate.

The term "surfactant" includes, but is not limited to, polysorbates (Tween, such as polysorbate 20 and polysorbate 80), poloxamers (e.g., poloxamer 188), triton, sodium dodecyl sulfate (SDS), sodium lauryl sulfate; sodium octyl glycoside, lauryl sulfobetaine, myristyl sulfobetaine, linoleyl sulfobetaine or stearyl sulfobetaine, lauryl sarcosine, myristyl sarcosine, linoleyl sarcosine or stearyl sarcosine, linoleyl betaine, myristyl betaine or cetyl betaine, lauryl amidopropyl betaine, cocoamidopropyl betaine, linoleamidopropyl betaine, myristamidopropyl betaine, palmitamidopropyl betaine or isostearamidopropyl betaine (e.g. laurylamidopropyl), myristamidopropyl dimethylamine, palmitoylaminopropyl dimethylamine or isostearamidopropyl dimethylamine, sodium methyl cocoyl taurate or disodium methyl oleyl taurate, polyethylene glycols, polypropylene glycols, copolymers of ethylene and propylene glycols (e.g., Pluronics, PF68, etc.) and the like. In some embodiments, the surfactant is polysorbate 80, also known as PS80 or Tween 80.

The "treatment" of a patient's disease means: (1) preventing a disease from developing in a patient who is prone to the disease or has not shown symptoms of the disease; (2) inhibiting the disease or symptoms of the disease, or preventing the progression of the disease; or (3) alleviating or eliminating the disease or symptoms of the disease, or making it regress.

The "stability" and "stable" herein mean that the antibody (including the antibody fragment thereof) undergoes no, or only minimal, aggregation, degradation, or fragmentation under given conditions of manufacture, preparation, transportation and/or storage in a formulation comprising the antibody. A "stable" formulation maintains biological activity in given conditions of manufacture, preparation, transportation and/or storage. The stability of the antibody can be evaluated by the extent of aggregation, degradation, fragmentation, or the like of the formulation as measured by technologies such as SEC-HPLC, IEC-HPLC, CE-SDS (NR), visual inspection and turbidity, insoluble particles, and detection of particle size by DLS.

The active ingredient in the formulation of the present disclosure is an anti-IL-4Rα antibody, including but not limited to monoclonal antibodies, chimeric antibodies, dAbs (domain antibodies), single-chain antibodies, Fab, Fab- and F(ab')₂ fragments, Fv, scFv, and Fab expression libraries. In some embodiments, the anti-IL-4Rα antibody is acquired by culturing a cell expressing the anti-IL-4Rα antibody and purifying. In some embodiments, the amino acid sequence of the heavy chain of the anti-IL-4Rα antibody is set forth in SEQ ID NO: 3, and the amino acid sequence of the light chain is set forth in SEQ ID NO: 4. In some embodiments, the anti-IL-4Rα antibody is dupilumab (e.g., Dupixent^{®} or biosimilars thereof).

In the following examples, reagents and instruments used are those conventional in the art and commercially available unless otherwise specified; the methods used are conventional in the art, and those skilled in the art can undoubtedly implement the methods and obtain the corresponding results according to the content of the examples.

The anti-IL-4Rα antibody used in the following examples is dupilumab, comprising 2 heavy chains with identical sequences and 2 light chains with identical sequences. The amino acid sequence of the heavy chain variable region is set forth in SEQ ID NO: 1, the amino acid sequence of the heavy chain is set forth in SEQ ID NO: 3, the amino acid sequence of the light chain variable region is set forth in SEQ ID NO: 2, and the amino acid sequence of the light chain is set forth in SEQ ID NO: 4.

The amino acid sequence of the heavy chain variable region is as follows:



 (SEQ ID NO: 1)

The amino acid sequence of the light chain variable region is as follows:


 (SEQ ID NO: 2)

The amino acid sequence of the heavy chain is as follows:

EVQLVESGGGLEQPGGSLRLSCAGSGFTFRDYAMTWVRQAPGKGLEWVSSISGSGGNTY

YADSVKGRFTISRDNSKNTLYLQMNSLRAEDTAVYYCAKDRLSITIRPRYYGLDVWGQG

TTVTVSSASTKGPSVFPLAPCSRSTSESTAALGCLVKDYFPEPVTVSWNSGALTSGVHTFP

AVLQSSGLYSLSSVVTVPSSSLGTKTYTCNVDHKPSNTKVDKRVESKYGPPCPPCPAPEFL

GGPSVFLFPPKPKDTLMISRTPEVTCVVVDVSQEDPEVQFNWYVDGVEVHNAKTKPREE

QFNSTYRVVSVLTVLHQDWLNGKEYKCKVSNKGLPSSIEKTISKAKGQPREPQVYTLPPS

QEEMTKNQVSLTCLVKGFYPSDIAVEWESNGQPENNYKTTPPVLDSDGSFFLYSRLTVDK

SRWQEGNVFSCSVMHEALHNHYTQKSLSLSLGK (SEQ ID NO: 3)

The amino acid sequence of the light chain is as follows:

DIVMTQSPLSLPVTPGEPASISCRSSQSLLYSIGYNYLDWYLQKSGQSPQLLIYLGSNRASG
VPDRFSGSGSGTDFTLKISRVEAEDVGFYYCMQALQTPYTFGQGTKLEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 4)

The DNA sequences of the heavy and light chains of the antibody were cloned into an expression vector, which was then transfected into CHO cells. The anti-IL-4Ra antibody was obtained after cell culture and purification.

In the following examples, SEC-HPLC, IEC-HPLC, CE-SDS (NR), and other assays were performed according to conventional methods.

### Example 1: Preliminary pH and buffer screening study

According to the mass changes of the monomer, aggregates, fragments, and the like of the sample in the conditions of high temperature and light, the preliminary ranges of the buffer and pH suitable for the anti-IL-4Rα antibody were selected.

### 1 Procedures

The components and contents of the formulations are shown in Table 1. The concentrations of the formulations are the theoretical concentrations, and the actual content of the components may be within a certain range, such as ±10% or ±5% of the theoretical concentration. The experimental conditions and the detection items are shown in Table 2.

**Table 1. Formulation design**

| Formulation No. | Buffer | Stabilizer | Surfactant | Antibody concentration (mg/mL) | pH |
|---|---|---|---|---|---|
| 1-1 | Histidine, 20 mM | | | 50 | 5.3 |
| 1-2 | | | | 50 | 5.6 |
| 1-3 | | | | 50 | 5.9 |
| 1-4 | | | | 50 | 6.2 |
| 1-5 | | Sucrose, 5% | Polysorbate 80 (PS80), 0.2% | 50 | 6.5 |
| 1-6 | Citric acid, 20 mM | | | 50 | 5.3 |
| 1-7 | | | | 50 | 5.6 |
| 1-8 | | | | 50 | 5.9 |
| 1-9 | | | | 50 | 6.2 |
| 1-10 | | | | 50 | 6.5 |

**Table 2. Experimental conditions and detection items**

| Experimental condition | Sampling time | Test item |
|---|---|---|
| light (4500 ± 500 lx, 25 ± 2 °C) | 0 day (d), 7 d, 14 d | SEC-HPLC, IEC-HPLC, CE-SDS(NR) |
| High temperature (away from light, 40 ± 2 °C) | 0 d, 1 week (W), 2 W, 3 W, 4 W | |

### 2 Results

### 2.1 light test

**Table 3. Detection results for samples under light (4500 ± 500 lx, 25 ± 2 °C)**

| Test item | | SEC-HPLC (%) | | | | CE-SDS(NR) (%) | | | |
|---|---|---|---|---|---|---|---|---|---|
| Sampling time | Formulation No. | Aggregate | Monomer | Fragment | | Fragment | HHL | Main peak | Aggregate |
| 0 d | 1-1 | 2.92 | 96.67 | 0.40 | | 1.99 | 0.55 | 97.29 | 0.72 |
| | 1-2 | 2.86 | 96.75 | 0.39 | | 2.07 | 0.54 | 97.14 | 0.79 |
| | 1-3 | 2.90 | 96.72 | 0.38 | | 2.12 | 0.58 | 97.17 | 0.71 |
| | 1-4 | 2.94 | 96.68 | 0.38 | | 2.00 | 0.59 | 97.29 | 0.71 |
| | 1-5 | 2.99 | 96.64 | 0.37 | | 1.98 | 0.60 | 97.29 | 0.73 |
| | 1-6 | 2.99 | 96.68 | 0.33 | | 1.86 | 0.57 | 97.52 | 0.62 |
| | 1-7 | 3.05 | 96.61 | 0.33 | | 2.05 | 0.64 | 97.19 | 0.76 |
| | 1-8 | 3.10 | 96.56 | 0.34 | | 2.01 | 0.55 | 97.27 | 0.72 |
| | 1-9 | 3.11 | 96.55 | 0.33 | | 2.06 | 0.56 | 97.22 | 0.72 |
| | 1-10 | 3.13 | 96.56 | 0.32 | | 2.10 | 0.59 | 97.12 | 0.78 |
| 7 d | 1-1 | 4.24 | 95.37 | 0.39 | | 3.68 | 1.73 | 95.18 | 1.14 |
| | 1-2 | 4.57 | 95.04 | 0.39 | | 4.13 | 1.97 | 94.53 | 1.34 |
| | 1-3 | 4.65 | 94.92 | 0.43 | | 3.80 | 1.70 | 94.91 | 1.29 |
| | 1-4 | 5.12 | 94.49 | 0.39 | | 3.70 | 1.62 | 94.78 | 1.52 |
| | 1-5 | 5.22 | 94.39 | 0.39 | | 3.52 | 1.56 | 94.92 | 1.56 |
| | 1-6 | 6.46 | 93.09 | 0.45 | | 4.69 | 2.26 | 93.52 | 1.79 |
| | 1-7 | 6.46 | 93.12 | 0.42 | | 4.16 | 1.86 | 94.07 | 1.77 |
| | 1-8 | 7.56 | 92.03 | 0.41 | | 4.30 | 1.98 | 93.44 | 2.26 |
| | 1-9 | 8.67 | 90.93 | 0.40 | | 4.00 | 1.80 | 92.99 | 3.01 |
| | 1-10 | 10.44 | 89.16 | 0.40 | | 3.61 | 1.79 | 92.37 | 4.02 |
| 14 d | 1-1 | 5.30 | 94.25 | 0.46 | | 5.03 | 2.42 | 93.52 | 1.45 |
| | 1-2 | 5.88 | 93.63 | 0.50 | | 5.43 | 2.58 | 92.73 | 1.84 |
| | 1-3 | 5.97 | 93.56 | 0.47 | | 4.99 | 2.27 | 93.31 | 1.70 |
| | 1-4 | 6.99 | 92.56 | 0.46 | | 4.89 | 2.33 | 92.84 | 2.27 |
| | 1-5 | 6.72 | 92.87 | 0.41 | | 4.30 | 1.92 | 93.72 | 1.98 |
| | 1-6 | 8.09 | 91.35 | 0.56 | | 5.83 | 2.74 | 92.09 | 2.08 |
| | 1-7 | 10.10 | 89.31 | 0.59 | | 6.89 | 3.07 | 90.36 | 2.75 |
| | 1-8 | 10.83 | 88.62 | 0.54 | | 6.44 | 2.78 | 90.52 | 3.04 |
| | 1-9 | 11.65 | 87.86 | 0.49 | | 5.61 | 2.21 | 90.55 | 3.84 |
| | 1-10 | 13.79 | 85.76 | 0.45 | | 4.71 | 1.98 | 90.01 | 5.28 |

As can be seen from the data in Table 3, in the same buffer system after 14 days of light, a lower pH resulted in better stability; at the same pH, the main peak of CE-SDS (NR) in the histidine buffer system was higher than that of citric acid, and the CE-SDS (NR) fragment and aggregate were all lower than those of citric acid. In general, the stability of the formulation containing histidine is better than that of the formulation containing citric acid.

### 2.2 High-temperature test

**Table 4. Detection results in samples at high temperature (away from light, 40 ± 2 °C)**

| Test item | | SEC-HPLC (%) | | | IEC-HPLC (%) | | | CE-SDS(NR) (%) | | | |
|---|---|---|---|---|---|---|---|---|---|---|---|
| Sampling time | Formulation No. | Aggregate | Monomer | Fragment | Acidic variant | Main peak | Basic variant | Fragment | HHL | Main peak | Aggregate |
| 0 d | 1-1 | 2.92 | 96.67 | 0.40 | 16.62 | 63.10 | 20.28 | 1.99 | 0.55 | 97.29 | 0.72 |
| | 1-2 | 2.86 | 96.75 | 0.39 | 16.39 | 63.22 | 20.39 | 2.07 | 0.54 | 97.14 | 0.79 |
| | 1-3 | 2.90 | 96.72 | 0.38 | 16.72 | 62.97 | 20.32 | 2.12 | 0.58 | 97.17 | 0.71 |
| | 1-4 | 2.94 | 96.68 | 0.38 | 17.05 | 62.66 | 20.29 | 2.00 | 0.59 | 97.29 | 0.71 |
| | 1-5 | 2.99 | 96.64 | 0.37 | 16.57 | 63.24 | 20.18 | 1.98 | 0.60 | 97.29 | 0.73 |
| | 1-6 | 2.99 | 96.68 | 0.33 | 16.41 | 63.42 | 20.17 | 1.86 | 0.57 | 97.52 | 0.62 |
| | 1-7 | 3.05 | 96.61 | 0.33 | 16.72 | 63.26 | 20.02 | 2.05 | 0.64 | 97.19 | 0.76 |
| | 1-8 | 3.10 | 96.56 | 0.34 | 16.71 | 62.90 | 20.39 | 2.01 | 0.55 | 97.27 | 0.72 |
| | 1-9 | 3.11 | 96.55 | 0.33 | 16.54 | 63.08 | 20.38 | 2.06 | 0.56 | 97.22 | 0.72 |
| | 1-10 | 3.13 | 96.56 | 0.32 | 16.73 | 62.87 | 20.39 | 2.10 | 0.59 | 97.12 | 0.78 |
| 1 W | 1-1 | 2.92 | 96.54 | 0.54 | 16.56 | 62.21 | 21.23 | 2.57 | 0.84 | 96.76 | 0.67 |
| | 1-2 | 2.94 | 96.55 | 0.51 | 17.00 | 62.39 | 20.61 | 2.69 | 0.89 | 96.60 | 0.71 |
| | 1-3 | 3.08 | 96.45 | 0.47 | 18.21 | 61.64 | 20.15 | 2.48 | 0.79 | 96.72 | 0.80 |
| | 1-4 | 3.16 | 96.43 | 0.41 | 19.55 | 60.31 | 20.14 | 2.50 | 0.72 | 96.75 | 0.75 |
| | 1-5 | 3.28 | 96.34 | 0.38 | 19.92 | 60.19 | 19.90 | 2.33 | 0.64 | 96.90 | 0.77 |
| | 1-6 | 3.52 | 95.84 | 0.64 | 18.45 | 61.12 | 20.43 | 2.88 | 0.74 | 96.39 | 0.73 |
| | 1-7 | 3.33 | 96.15 | 0.52 | 19.12 | 60.69 | 20.19 | 2.64 | 0.81 | 96.55 | 0.81 |
| | 1-8 | 3.34 | 96.21 | 0.45 | 19.37 | 60.79 | 19.84 | 2.54 | 0.72 | 96.69 | 0.77 |
| | 1-9 | 3.41 | 96.20 | 0.39 | 19.71 | 60.36 | 19.93 | 2.36 | 0.62 | 96.81 | 0.83 |
| | 1-10 | 3.53 | 96.10 | 0.37 | 20.89 | 59.51 | 19.60 | 2.36 | 0.60 | 96.73 | 0.91 |
| 2 W | 1-1 | 3.14 | 96.17 | 0.69 | 17.77 | 60.79 | 21.43 | 4.16 | 1.71 | 95.18 | 0.66 |
| | 1-2 | 3.07 | 96.33 | 0.59 | 18.89 | 59.90 | 21.20 | 3.47 | 1.20 | 95.71 | 0.82 |
| | 1-3 | 3.17 | 96.32 | 0.51 | 20.45 | 59.07 | 20.48 | 3.00 | 0.95 | 96.00 | 1.00 |
| | 1-4 | 3.28 | 96.30 | 0.42 | 22.90 | 57.37 | 19.74 | 2.82 | 0.82 | 96.31 | 0.87 |
| | 1-5 | 3.41 | 96.17 | 0.42 | 23.37 | 57.55 | 19.09 | 2.91 | 0.67 | 96.33 | 0.76 |
| | 1-6 | 3.99 | 95.15 | 0.86 | 22.51 | 58.33 | 19.16 | 2.82 | 0.66 | 96.24 | 0.94 |
| | 1-7 | 3.45 | 95.89 | 0.65 | 22.65 | 57.32 | 20.04 | 3.27 | 0.92 | 96.03 | 0.70 |
| | 1-8 | 3.35 | 96.16 | 0.49 | 22.11 | 58.35 | 19.55 | 2.86 | 0.64 | 96.25 | 0.89 |
| | 1-9 | 3.48 | 96.13 | 0.39 | 21.98 | 57.42 | 20.60 | 3.51 | 1.22 | 95.51 | 0.98 |
| | 1-10 | 3.58 | 96.05 | 0.36 | 25.19 | 56.13 | 18.68 | 3.06 | 0.68 | 95.79 | 1.15 |
| 3 W | 1-1 | 3.54 | 93.90 | 2.56 | 19.68 | 58.63 | 21.69 | 5.39 | 2.17 | 93.80 | 0.81 |
| | 1-2 | 3.32 | 95.95 | 0.72 | 20.67 | 58.15 | 21.18 | 4.24 | 1.77 | 95.09 | 0.67 |
| | 1-3 | 3.33 | 96.08 | 0.59 | 23.36 | 56.53 | 20.10 | 3.19 | 0.97 | 96.03 | 0.78 |
| | 1-4 | 3.47 | 96.09 | 0.44 | 27.44 | 53.84 | 18.73 | 3.04 | 0.79 | 96.07 | 0.89 |
| | 1-5 | 3.55 | 96.02 | 0.43 | 27.98 | 53.57 | 18.45 | 2.93 | 0.61 | 96.14 | 0.93 |
| | 1-6 | 4.38 | 92.38 | 3.25 | 25.20 | 54.58 | 20.22 | 6.44 | 1.87 | 92.80 | 0.76 |
| | 1-7 | 3.60 | 95.62 | 0.78 | 24.50 | 55.79 | 19.70 | 3.63 | 0.94 | 95.57 | 0.80 |
| | 1-8 | 3.53 | 95.87 | 0.60 | 26.73 | 54.34 | 18.92 | 3.30 | 0.82 | 95.87 | 0.83 |
| | 1-9 | 3.65 | 95.94 | 0.41 | 28.93 | 52.73 | 18.34 | 3.28 | 0.65 | 95.68 | 1.04 |
| | 1-10 | 3.76 | 95.86 | 0.38 | 34.28 | 48.13 | 17.59 | 3.21 | 0.66 | 95.84 | 0.95 |
| 4 W | 1-1 | 4.03 | 93.75 | 2.21 | NA | NA | NA | 7.59 | 3.42 | 91.58 | 0.83 |
| | 1-2 | 3.58 | 95.63 | 0.79 | NA | NA | NA | 5.69 | 2.33 | 93.41 | 0.90 |
| | 1-3 | 3.50 | 95.88 | 0.62 | NA | NA | NA | 3.91 | 1.29 | 95.04 | 1.05 |
| | 1-4 | 3.52 | 96.03 | 0.45 | NA | NA | NA | 3.67 | 0.96 | 95.24 | 1.09 |
| | 1-5 | 3.61 | 95.99 | 0.41 | NA | NA | NA | 3.65 | 0.89 | 95.15 | 1.20 |
| | 1-6 | 4.65 | 92.32 | 3.03 | NA | NA | NA | 8.47 | 2.63 | 90.72 | 0.81 |
| | 1-7 | 3.69 | 95.48 | 0.83 | NA | NA | NA | 4.82 | 1.09 | 94.20 | 0.98 |
| | 1-8 | 3.63 | 95.79 | 0.58 | NA | NA | NA | 3.94 | 1.13 | 95.04 | 1.02 |
| | 1-9 | 3.69 | 95.89 | 0.42 | NA | NA | NA | 3.48 | 0.76 | 95.47 | 1.05 |
| | 1-10 | 3.78 | 95.85 | 0.37 | NA | NA | NA | 3.71 | 0.71 | 95.08 | 1.21 |

As can be seen from the SEC-HPLC detection results, after 4 weeks of high-temperature storage, the antibody exhibited good stability in histidine and citric acid buffer systems at pH 5.6-6.5, with no significant differences between the two buffer systems.

As can be seen from the IEC-HPLC detection results, in the same buffer system, in the course of the high-temperature storage, a lower pH resulted in slower main peak decrease and acidic variant increase and thus better stability, with both buffer systems showing the best stability at pH 5.3-5.9; at the same pH, the histidine buffer system exhibited better stability than the citric acid buffer system.

As can be seen from the CE-SDS (NR) detection results, after 4 weeks of high-temperature storage, the antibody exhibited no significant differences in stability between the histidine and citric acid buffer systems at pH 5.6-6.5, with the range of pH 5.9-6.5 the most preferred and pH 5.6 the second most preferred.

By synthesizing the results of light and high-temperature tests, it was found that the light has a greater effect on the samples than the high temperature. The samples should be carefully protected from long-term exposure to the light condition, and the histidine buffer system is generally more stable than the citric acid buffer system. In summary, histidine buffer with a pH range of 5.6-6.2 was tentatively determined as the buffer.

### Example 2: Stabilizer screening study

Based on the 20 mM histidine buffer system at pH 5.9, the stabilizers were screened to explore the trend of change in the quality of the anti-IL-4Rα antibody with different compositions, so as to determine a stabilizer with good protective effects.

### 1 Procedures

The components and contents of the formulations are shown in Table 5. The experimental conditions and the detection items are shown in Table 6.

**Table 5. Formulation design**

| Formulation No. | Buffer | Stabilizer | Surfactant | Antibody concentration (mg/mL) | pH |
|---|---|---|---|---|---|
| 2-1 | Histidine, 20 mM | Sucrose, 8.0% | | 50 | 5.9 |
| 2-2 | | Trehalose dihydrate, 8.5% | | 50 | 5.9 |
| 2-3 | | Glycine, 2.2% | | 50 | 5.9 |
| 2-4 | | None | | 50 | 5.9 |
| 2-5 | | Mannitol, 4.5% | | 50 | 5.9 |
| 2-6 | | Arginine hydrochloride, 5.0% | 0.2% PS80 | 50 | 5.9 |
| 2-7 | | Sucrose, 3.0% + arginine hydrochloride, 5.0% | | 50 | 5.9 |
| 2-8 (control formulation) | Histidine, 20 mM + sodium acetate, 12.5 mM | Sucrose, 5.0% + arginine hydrochloride, 25 mM | | 50 | 5.9 |
| 2-9 | Histidine, 20 mM | Proline, 2.5% | | 50 | 5.9 |

**Table 6. Experimental conditions and detection items**

| Experimental condition | Sampling time | Test item |
|---|---|---|
| light (4500 ± 500 lx, 25 + 2 °C) | 0 d, 6 d, 12 d | SEC-HPLC, IEC-HPLC, CE-SDS(NR) |
| High temperature (away from light, 40 ± 2 °C) | 0 d, 1 W, 2 W, 3 W, 4 W | |

### 2 Results

### 2.1 light test

**Table 7. Detection results in samples under light (4500 ± 500 lx, 25 ± 2 °C)**

| Test item | | SEC-HPLC (%) | | | CE-SDS(NR) (%) | | | |
|---|---|---|---|---|---|---|---|---|
| Sampling time | Formulation No. | Aggregate | Monomer | Fragment | Fragment | HHL | Main peak | Aggregate |
| 0 d | 2-1 | 3.35 | 96.40 | 0.24 | 2.69 | 1.14 | 96.35 | 0.96 |
| | 2-2 | 3.41 | 96.36 | 0.23 | 2.84 | 1.26 | 96.21 | 0.95 |
| | 2-3 | 3.36 | 96.41 | 0.23 | 2.85 | 1.22 | 96.16 | 0.99 |
| | 2-4 | 3.41 | 96.35 | 0.25 | 2.68 | 1.07 | 96.29 | 1.03 |
| | 2-5 | 3.42 | 96.34 | 0.24 | 2.69 | 1.07 | 96.27 | 1.04 |
| | 2-6 | 3.37 | 96.38 | 0.25 | 2.72 | 1.08 | 96.15 | 1.13 |
| | 2-7 | 3.37 | 96.40 | 0.23 | 2.64 | 1.11 | 96.46 | 0.90 |
| | 2-8 | 3.39 | 96.38 | 0.23 | 2.74 | 1.20 | 96.21 | 1.05 |
| | 2-9 | 3.33 | 96.44 | 0.23 | 2.63 | 1.11 | 96.45 | 0.92 |
| 6 d | 2-1 | 6.02 | 93.67 | 0.31 | 6.49 | 3.30 | 91.13 | 2.38 |
| | 2-2 | 6.04 | 93.67 | 0.29 | 5.90 | 3.02 | 91.67 | 2.43 |
| | 2-3 | 5.34 | 94.35 | 0.30 | 6.41 | 3.39 | 91.53 | 2.06 |
| | 2-4 | 6.28 | 93.44 | 0.28 | 6.03 | 3.08 | 91.31 | 2.66 |
| | 2-5 | 6.01 | 93.71 | 0.28 | 6.30 | 3.29 | 91.21 | 2.49 |
| | 2-6 | 4.96 | 94.71 | 0.33 | 6.33 | 3.13 | 91.97 | 1.70 |
| | 2-7 | 5.01 | 94.68 | 0.31 | 6.32 | 3.11 | 92.07 | 1.61 |
| | 2-8 | 5.65 | 94.05 | 0.30 | 6.25 | 3.12 | 91.67 | 2.08 |
| | 2-9 | 5.50 | 94.20 | 0.29 | 5.75 | 2.89 | 92.06 | 2.19 |
| 12 d | 2-1 | 7.51 | 92.11 | 0.39 | 8.13 | 4.10 | 89.30 | 2.57 |
| | 2-2 | 8.61 | 90.96 | 0.42 | 8.84 | 4.53 | 88.10 | 3.06 |
| | 2-3 | 6.92 | 92.65 | 0.43 | 8.13 | 4.16 | 89.87 | 2.00 |
| | 2-4 | 8.83 | 90.75 | 0.42 | 8.47 | 4.31 | 88.16 | 3.37 |
| | 2-5 | 8.15 | 91.43 | 0.41 | 7.95 | 4.05 | 89.30 | 2.75 |
| | 2-6 | 6.36 | 93.14 | 0.50 | 9.43 | 4.65 | 88.79 | 1.78 |
| | 2-7 | 6.17 | 93.41 | 0.42 | 8.66 | 4.27 | 89.48 | 1.86 |
| | 2-8 | 7.14 | 92.46 | 0.40 | 8.64 | 4.37 | 89.11 | 2.25 |
| | 2-9 | 6.94 | 92.66 | 0.40 | 8.26 | 4.13 | 89.50 | 2.24 |

As can be seen from Table 7, after 12 days of light, Formulations 2-6 and 2-7 exhibited the highest monomer and the lowest aggregate by SEC-HPLC and thus the best stability, while Formulations 2-3 and 2-9 exhibited relatively good stability and Formulations 2-2 and 2-5 exhibited relatively poor stability. After 12 days of light, the formulations exhibited no significant differences in CE-SDS (NR) main peak.

### 2.2 High-temperature test

**Table 8. Detection results in samples at high temperature (away from light, 40 ± 2 °C)**

| Test item | | SEC-HPLC (%) | | | IEC-HPLC (%) | | | CE-SDS(NR) (%) | | | |
|---|---|---|---|---|---|---|---|---|---|---|---|
| Sampling time | Formulation No. | Aggregate | Monomer | Fragment | Acidic variant | Main peak | Basic variant | Fragment | HHL | Main peak | Aggregate |
| 0 d | 2-1 | 3.35 | 96.40 | 0.24 | 23.96 | 57.90 | 18.14 | 2.69 | 1.14 | 96.35 | 0.96 |
| | 2-2 | 3.41 | 96.36 | 0.23 | 24.01 | 57.51 | 18.48 | 2.84 | 1.26 | 96.21 | 0.95 |
| | 2-3 | 3.36 | 96.41 | 0.23 | 24.18 | 57.21 | 18.62 | 2.85 | 1.22 | 96.16 | 0.99 |
| | 2-4 | 3.41 | 96.35 | 0.25 | 24.62 | 57.42 | 17.96 | 2.68 | 1.07 | 96.29 | 1.03 |
| | 2-5 | 3.42 | 96.34 | 0.24 | 24.06 | 57.24 | 18.70 | 2.69 | 1.07 | 96.27 | 1.04 |
| | 2-6 | 3.37 | 96.38 | 0.25 | 24.08 | 57.64 | 18.28 | 2.72 | 1.08 | 96.15 | 1.13 |
| | 2-7 | 3.37 | 96.40 | 0.23 | 24.31 | 57.93 | 17.76 | 2.64 | 1.11 | 96.46 | 0.90 |
| | 2-8 | 3.39 | 96.38 | 0.23 | 24.15 | 57.53 | 18.31 | 2.74 | 1.20 | 96.21 | 1.05 |
| | 2-9 | 3.33 | 96.44 | 0.23 | 24.28 | 57.30 | 18.42 | 2.63 | 1.11 | 96.45 | 0.92 |
| 1W | 2-1 | 3.20 | 96.49 | 0.30 | 27.13 | 53.98 | 18.89 | 2.97 | 1.17 | 95.93 | 1.10 |
| | 2-2 | 3.19 | 96.51 | 0.30 | 26.84 | 54.29 | 18.87 | 3.02 | 1.17 | 95.89 | 1.09 |
| | 2-3 | 3.52 | 96.16 | 0.33 | 29.59 | 53.04 | 17.37 | 3.31 | 1.25 | 95.55 | 1.14 |
| | 2-4 | 3.27 | 96.44 | 0.29 | 26.93 | 54.47 | 18.60 | 2.98 | 1.18 | 95.99 | 1.03 |
| | 2-5 | 3.21 | 96.49 | 0.30 | 26.78 | 55.07 | 18.15 | 2.92 | 1.09 | 96.07 | 1.01 |
| | 2-6 | 3.43 | 96.26 | 0.31 | 27.04 | 54.70 | 18.26 | 3.13 | 1.23 | 95.65 | 1.22 |
| | 2-7 | 3.27 | 96.42 | 0.31 | 26.91 | 54.71 | 18.38 | 3.15 | 1.22 | 95.74 | 1.11 |
| | 2-8 | 3.28 | 96.42 | 0.29 | 27.21 | 54.60 | 18.18 | 3.02 | 1.13 | 96.01 | 0.97 |
| | 2-9 | 3.07 | 96.66 | 0.27 | 26.23 | 55.61 | 18.16 | 2.86 | 1.07 | 96.06 | 1.08 |
| 2W | 2-1 | 3.13 | 96.57 | 0.30 | 26.50 | 56.50 | 17.00 | 3.73 | 1.60 | 95.41 | 0.86 |
| | 2-2 | 3.20 | 96.49 | 0.30 | 26.81 | 56.46 | 16.73 | 3.80 | 1.66 | 95.35 | 0.85 |
| | 2-3 | 3.33 | 96.35 | 0.32 | 27.85 | 55.92 | 16.23 | 4.09 | 1.76 | 95.01 | 0.90 |
| | 2-4 | 3.27 | 96.44 | 0.29 | 26.95 | 56.82 | 16.24 | 3.75 | 1.69 | 95.34 | 0.91 |
| | 2-5 | 3.21 | 96.48 | 0.31 | 26.12 | 57.82 | 16.06 | 3.84 | 1.71 | 95.23 | 0.93 |
| | 2-6 | 3.41 | 96.29 | 0.30 | 24.43 | 58.83 | 16.73 | 4.02 | 1.58 | 94.98 | 1.00 |
| | 2-7 | 3.22 | 96.45 | 0.33 | 26.15 | 57.30 | 16.55 | 3.97 | 1.62 | 95.11 | 0.92 |
| | 2-8 | 3.19 | 96.50 | 0.30 | 26.87 | 56.42 | 16.72 | 3.51 | 1.49 | 95.61 | 0.88 |
| | 2-9 | 3.02 | 96.71 | 0.26 | 27.92 | 55.93 | 16.15 | 3.87 | 1.60 | 95.26 | 0.87 |
| 3W | 2-1 | 3.55 | 95.98 | 0.47 | 30.62 | 51.22 | 18.15 | 3.77 | 1.46 | 95.17 | 1.06 |
| | 2-2 | 3.69 | 95.85 | 0.45 | 29.01 | 52.68 | 18.31 | 4.09 | 1.62 | 94.92 | 0.99 |
| | 2-3 | 3.82 | 95.69 | 0.49 | 30.88 | 51.73 | 17.39 | 4.38 | 1.69 | 94.56 | 1.06 |
| | 2-4 | 3.78 | 95.75 | 0.47 | 28.24 | 53.36 | 18.41 | 4.10 | 1.51 | 94.77 | 1.13 |
| | 2-5 | 3.68 | 95.86 | 0.46 | 29.58 | 52.10 | 18.33 | 4.13 | 1.51 | 94.76 | 1.11 |
| | 2-6 | 3.99 | 95.54 | 0.47 | 32.67 | 48.79 | 18.54 | 4.56 | 1.54 | 94.31 | 1.13 |
| | 2-7 | 3.77 | 95.76 | 0.46 | 32.68 | 48.62 | 18.70 | 4.52 | 1.64 | 94.43 | 1.05 |
| | 2-8 | 3.67 | 95.88 | 0.45 | 32.65 | 48.48 | 18.87 | 4.28 | 1.64 | 94.75 | 0.97 |
| | 2-9 | 3.45 | 96.10 | 0.45 | 28.46 | 53.37 | 18.16 | 4.17 | 1.59 | 94.8 | 1.03 |
| 4W | 2-1 | 3.63 | 95.91 | 0.46 | 33.86 | 46.67 | 19.47 | 4.70 | 1.75 | 94.25 | 1.05 |
| | 2-2 | 3.75 | 95.79 | 0.46 | 34.44 | 46.68 | 18.88 | 4.77 | 1.72 | 94.17 | 1.06 |
| | 2-3 | 3.93 | 95.61 | 0.46 | 35.78 | 46.11 | 18.11 | 5.17 | 1.95 | 93.78 | 1.05 |
| | 2-4 | 3.80 | 95.75 | 0.45 | 31.99 | 49.71 | 18.30 | 4.79 | 1.79 | 94.28 | 0.93 |
| | 2-5 | 3.69 | 95.84 | 0.47 | 31.79 | 49.55 | 18.67 | 4.91 | 1.80 | 94.12 | 0.97 |
| | 2-6 | 4.09 | 95.46 | 0.45 | 33.32 | 46.96 | 19.73 | 6.76 | 2.48 | 92.25 | 0.99 |
| | 2-7 | 3.79 | 95.73 | 0.47 | 33.06 | 46.93 | 20.01 | 5.04 | 1.81 | 93.96 | 1.00 |
| | 2-8 | 3.67 | 95.87 | 0.46 | 34.54 | 46.72 | 18.74 | 4.80 | 1.68 | 94.29 | 0.91 |
| | 2-9 | 3.50 | 96.05 | 0.45 | 30.60 | 48.36 | 21.03 | 4.77 | 1.70 | 94.34 | 0.89 |

As can be seen from the data in Table 8, after 4 weeks of high-temperature storage, the SEC-HPLC detection results of the formulations exhibited no significant differences, and the SEC-HPLC aggregate of the proline formulation exhibited the slowest increase compared to the other formulations, suggesting better stability. After 4 weeks of high-temperature storage, Formulation 2-4 without stabilizers, Formulation 2-5 with mannitol, and Formulation 2-9 with proline exhibited slightly higher main peaks and slightly lower acidic variants by IEC-HPLC, suggesting better stability than the other formulations. After 4 weeks of high-temperature storage, Formulation 2-6 with arginine hydrochloride exhibited the lowest main peak and the highest fragment by CE-SDS (NR) and thus the poorest stability, while the other formulations exhibited no significant differences in the CE-SDS (NR) detection results.

As can be seen from the light and high-temperature test results, the formulations with trehalose and mannitol have the poorest stability, the formulation with arginine hydrochloride alone has relatively poor stability, the formulation with both arginine hydrochloride and sucrose has good stability, while the formulation with proline has relatively good stability in general.

### Example 3: PS80 degradation study

The effects of different buffer systems and different antioxidants and metal chelating agents on the degradation of PS80 in the formulations were explored.

### 1 Procedures

The components and contents of the formulations are shown in Table 9. The experimental conditions and the detection items are shown in Table 10.

**Table 9. Formulation design**

| Formulation No. | Buffer | Stabilizer | Surfactant | Concentration of anti-IL-4Rα antibody protein | pH |
|---|---|---|---|---|---|
| 3-1 | Histidine, 20 mM | Arginine hydrochloride, 110 mM + sucrose, 1.5% | 0.1% PS80 | | |
| 3-2 | | Arginine hydrochloride, 110 mM + sucrose, 1.5% + ethylenediamine tetraacetic acid (EDTA), 0.05 mM | | | |
| 3-3 | | Arginine hydrochloride, 110 mM + sucrose, 1.5% + methionine, 10 mM | | | |
| 3-4 | Histidine, 20 mM + citric acid, 20 mM | Arginine hydrochloride, 110 mM + sucrose, 1.5% | | 175 mg/mL | 5.9±0.3 |
| 3-5 | Histidine, 20 mM + acetic acid, 33 ± 5 mM | Proline, 2.5% + methionine, 10 mM | | | |
| 3-6 (control formulation) | Histidine, 20 mM + sodium acetate, 12.5 mM | Arginine hydrochloride, 50 mM + sucrose, 5% | 0.2% PS80 | | |

**Table 10. Experimental conditions and detection items**

| Experimental condition | Sampling time | Test item |
|---|---|---|
| light (4500 ± 500 lx, 25 ± 2 °C) | 0 d, 6 d, 12 d | PS80 content by SEC-HPLC, IEC-HPLC, CE-SDS (NR) |
| High temperature (away from light, 40 ± 2 °C) | 0 d, 2 W, 4W | |
| Accelerated (25 ± 2 °C) | 1 month (M), 2 M, 3 M | |

### 2 Results

### 2.1 Antibody concentration and physicochemical detection results

**Table 11. Antibody concentration and physicochemical detection results**

| Formulation No. | Protein concentration (mg/mL) | pH | Osmolality (mOsmol/kg) |
|---|---|---|---|
| 3-1 | 175.79 | 5.79 | 339 |
| 3-2 | 172.23 | 5.79 | 334 |
| 3-3 | 176.08 | 5.81 | 341 |
| 3-4 | 182.94 | 5.66 | 387 |
| 3-5 | 183.05 | 5.78 | 366 |
| 3-6 | 181.24 | 5.77 | 374 |

As can be seen from the data in Table 11, Formulation 3-4, Formulation 3-5, and control Formulation 3-6 all exhibited high osmolality.

### 2.2 light test

**Table 12. Detection results in samples under light (4500 ± 500 lx, 25 ± 2 °C)**

| Test item | | SEC-HPLC (%) | | | CE-SDS(NR) (%) | | | |
|---|---|---|---|---|---|---|---|---|
| Sampling time | Formulation No. | Aggregate | Monomer | Fragment | Fragment | HHL | Main peak | Aggregate |
| 0 d | 3-1 | 1.26 | 98.53 | 0.21 | 1.94 | 0.72 | 97.49 | 0.57 |
| | 3-2 | 1.24 | 98.59 | 0.17 | 1.89 | 0.71 | 97.45 | 0.66 |
| | 3-3 | 1.21 | 98.60 | 0.19 | 1.85 | 0.71 | 97.55 | 0.60 |
| | 3-4 | 1.25 | 98.63 | 0.12 | 1.78 | 0.68 | 97.59 | 0.63 |
| | 3-5 | 1.33 | 98.47 | 0.20 | 1.94 | 0.72 | 97.46 | 0.60 |
| | 3-6 | 1.26 | 98.54 | 0.20 | 1.91 | 0.72 | 97.51 | 0.58 |
| 6 d | 3-1 | 1.55 | 98.18 | 0.28 | 2.24 | 0.91 | 97.01 | 0.75 |
| | 3-2 | 1.62 | 98.16 | 0.22 | 2.17 | 0.92 | 97.07 | 0.76 |
| | 3-3 | 1.59 | 98.16 | 0.26 | 2.31 | 0.91 | 96.85 | 0.84 |
| | 3-4 | 1.54 | 98.29 | 0.16 | 2.17 | 0.90 | 97.18 | 0.65 |
| | 3-5 | 1.62 | 98.18 | 0.2 | 2.22 | 0.89 | 97.02 | 0.76 |
| | 3-6 | 1.74 | 98.02 | 0.24 | 2.29 | 0.94 | 96.92 | 0.79 |
| 12 d | 3-1 | 1.55 | 98.23 | 0.22 | 3.02 | 1.31 | 96.22 | 0.76 |
| | 3-2 | 1.64 | 98.20 | 0.16 | 2.94 | 1.24 | 96.29 | 0.77 |
| | 3-3 | 1.69 | 98.07 | 0.23 | 3.05 | 1.26 | 96.14 | 0.81 |
| | 3-4 | 1.60 | 98.22 | 0.17 | 3.08 | 1.35 | 96.19 | 0.73 |
| | 3-5 | 1.61 | 98.25 | 0.15 | 2.91 | 1.19 | 96.40 | 0.69 |
| | 3-6 | 1.81 | 97.99 | 0.20 | 2.91 | 1.20 | 96.13 | 0.96 |

As can be seen from Table 12, after 12 days of light, no significant differences in SEC-HPLC detection results and CE-SDS (NR) detection results for the formulation samples were found.

**Table 13. Polysorbate 80 content assay results under light (4500 ± 500 lx, 25 ± 2 °C)**

| Formulation No. | PS80 (µg/mL) | | PS80 decrease rate after 12 d (%) |
|---|---|---|---|
| | 0 d | 12 d | |
| 3-1 | 1060 | 419 | 60 |
| 3-2 | 1027 | 401 | 61 |
| 3-3 | 1030 | 451 | 56 |
| 3-4 | 949 | 295 | 69 |
| 3-5 | 1043 | 882 | 15 |
| 3-6 | 1912 | 1083 | 43 |

As can be seen from the data in Table 13, the PS80 content of Formulation 3-5 decreased by about 15% after 12 days of light, indicating that Formulation 3-5 can effectively slow down the degradation of PS80 in the formulation under light.

### 2.3 High-temperature test

**Table 14. Detection results in samples at high temperature (away from light, 40 ± 2 °C)**

| Test item | | SEC-HPLC (%) | | | IEC-HPLC (%) | | | CE-SDS(NR) (%) | | | |
|---|---|---|---|---|---|---|---|---|---|---|---|
| Sampling time | Formulation No. | Aggregate | Monomer | Fragment | Acidic variant | Main peak | Basic variant | Fragment | HHL | Main peak | Aggregate |
| 0 d | 3-1 | 1.26 | 98.53 | 0.21 | 20.01 | 53.69 | 26.30 | 1.94 | 0.72 | 97.49 | 0.57 |
| | 3-2 | 1.24 | 98.59 | 0.17 | 20.44 | 54.47 | 25.09 | 1.89 | 0.71 | 97.45 | 0.66 |
| | 3-3 | 1.21 | 98.60 | 0.19 | 20.11 | 54.06 | 25.84 | 1.85 | 0.71 | 97.55 | 0.60 |
| | 3-4 | 1.25 | 98.63 | 0.12 | 20.50 | 53.77 | 25.74 | 1.78 | 0.68 | 97.59 | 0.63 |
| | 3-5 | 1.33 | 98.47 | 0.20 | 20.12 | 54.56 | 25.32 | 1.94 | 0.72 | 97.46 | 0.60 |
| | 3-6 | 1.26 | 98.54 | 0.20 | 20.91 | 53.58 | 25.51 | 1.91 | 0.72 | 97.51 | 0.58 |
| 2W | 3-1 | 1.67 | 98.05 | 0.28 | 22.52 | 51.11 | 26.37 | 3.00 | 1.05 | 96.23 | 0.77 |
| | 3-2 | 1.68 | 98.06 | 0.26 | 20.58 | 52.37 | 27.05 | 2.94 | 1.10 | 96.27 | 0.79 |
| | 3-3 | 1.65 | 98.12 | 0.22 | 22.69 | 50.43 | 26.89 | 3.13 | 1.16 | 96.13 | 0.74 |
| | 3-4 | 1.82 | 97.95 | 0.23 | 25.05 | 48.76 | 26.19 | 2.75 | 0.87 | 96.43 | 0.82 |
| | 3-5 | 1.77 | 98.03 | 0.20 | 24.39 | 49.43 | 26.18 | 2.92 | 1.08 | 96.25 | 0.83 |
| | 3-6 | 1.77 | 98.01 | 0.22 | 23.77 | 50.08 | 26.15 | 2.94 | 1.07 | 96.24 | 0.82 |
| 4W | 3-1 | 1.89 | 97.80 | 0.31 | 27.57 | 46.06 | 26.37 | 4.16 | 1.26 | 94.87 | 0.97 |
| | 3-2 | 1.82 | 97.91 | 0.28 | 27.21 | 46.89 | 25.91 | 4.38 | 1.64 | 94.75 | 0.87 |
| | 3-3 | 1.81 | 97.90 | 0.29 | 27.61 | 46.29 | 26.1 | 4.28 | 1.65 | 95.01 | 0.71 |
| | 3-4 | 2.29 | 97.52 | 0.19 | 32.43 | 42.85 | 24.72 | 3.84 | 1.08 | 95.15 | 1.01 |
| | 3-5 | 1.95 | 97.79 | 0.26 | 29.63 | 45.52 | 24.85 | 4.07 | 1.60 | 95.03 | 0.90 |
| | 3-6 | 1.98 | 97.76 | 0.25 | 28.88 | 45.56 | 25.56 | 3.99 | 1.51 | 95.12 | 0.89 |

As can be seen from the data in Table 14, after 4 weeks of high-temperature storage, the SEC-HPLC detection results of the formulation samples exhibited no significant differences. After 4 weeks of high-temperature storage, Formulation 3-4 with citric acid exhibited a relatively fast decrease in the main peak and a relatively fast increase in the acidic variants by IEC-HPLC and thus relatively poor stability, while the other formulation samples exhibited no significant differences in the IEC-HPLC detection results. After 4 weeks of high temperature storage, the formulations exhibited no significant differences in the CE-SDS (NR) detection results.

**Table 15. Polysorbate 80 content assay results at high temperature (away from light, 40 ± 2 °C)**

| Formulation No. | PS80 (µg/mL) | | PS80 decrease rate after 4 W (%) |
|---|---|---|---|
| | 0 d | 4W | |
| 3-1 | 1060 | 653 | 38 |
| 3-2 | 1027 | 387 | 62 |
| 3-3 | 1030 | 407 | 60 |
| 3-4 | 949 | 288 | 70 |
| 3-5 | 1043 | 822 | 21 |
| 3-6 | 1912 | 916 | 52 |

As can be seen from the data in Table 15, Formulation 3-5 is better than the other formulations in slowing down the degradation of PS80 in the formulation in high-temperature conditions.

In summary, the purity of the formulation samples with citric acid decreased fast under light and high-temperature storage, and the purity of the other formulation samples exhibited no significant differences. Formulation 3-5 with proline as the stabilizer exhibited the slowest PS80 degradation.

### Example 4: Formulation stability study

### 1 Procedures

The components and contents of the formulations are shown in Table 16. The experimental conditions and the detection items are shown in Table 17.

**Table 16. Formulation design**

| Formulation No. | Buffer | Stabilizer | Surfactant | Concentration of anti-IL-4Rα antibody | pH |
|---|---|---|---|---|---|
| 4-1 | Histidine, 20 mM | Arginine hydrochloride, 110 mM + sucrose, 1.5% | 0.2% PS80 | | |
| 4-2 | | Arginine hydrochloride, 110 mM + sucrose, 1.5% | Poloxamer 188, 0.05% | 175 mg/mL | 5.9±0.3 |
| 4-3 | | Proline, 217 mM (2.5%) + methionine, 10 mM | 0.2% PS80 | | |
| 4-4 | Histidine, 10 mM | Proline, 217 mM (2.5%) + methionine, 10 mM | 0.2% PS80 | | |
| 4-5 | Histidine, 20 mM + acetic acid, 31 ± 5 mM | Proline, 217 mM (2.5%) + methionine, 10 mM | 0.2% PS80 | | |
| 4-6 (control formulation) | Histidine, 20 mM + sodium acetate, 12.5 mM | Arginine hydrochloride, 50 mM + sucrose, 5% | 0.2% PS80 | | |

**Table 17. Experimental conditions and detection items**

| Experimental condition | Sampling time | Test item |
|---|---|---|
| light (4500 ± 500 lx, 25 ± 2 °C) | 0 d, 7 d, 14 d | |
| High temperature (away from light, 40 ± 2 °C) | 0 d, 2 W, 4W | PS80 content by SEC-HPLC, IEC-HPLC, CE-SDS (NR) |
| Accelerated (25 ± 2 °C) | 0 d, 1 M, 2 M, 3 M | |

### 2 Results

### 2.1 Detection results of viscosity and sliding force

**Table 18. Detection results of viscosity and sliding force of formulation samples**

| Formulation No. | Protein concentration (mg/mL) | Viscosity (cP) | Initiation force (N) | Max continuous force (N) | Average continuous force (N) |
|---|---|---|---|---|---|
| 4-1 | 184.83 | 9.99 | 8.74 | 10.02 | 9.31 |
| 4-2 | 185.54 | 9.78 | 8.96 | 10.81 | 9.90 |
| 4-3 | 180.12 | 15.02 | 13.32 | 14.83 | 14.29 |
| 4-4 | 175.34 | 14.34 | 11.67 | 12.41 | 12.06 |
| 4-5 | 181.57 | 14.83 | 11.60 | 12.31 | 11.98 |
| 4-6 | 179.59 | 11.74 | 10.54 | 11.45 | 11.02 |

| | | | | | |
|---|---|---|---|---|---|
| Note: In the sliding force test, the inner packaging material was a 2.25 mL pre-filled syringe (with a 27G1/2 syringe needle), and the loading volume was 1.14 mL/syringe. | | | | | |

As can be seen from the data in Table 18, Formulation 4-5 was slightly higher in viscosity than Formulation 4-6, while the sliding forces exhibited no significant differences. The viscosity and sliding force of the formulations in pre-filled syringes are within acceptable ranges.

### 2.2 Antibody concentration and physicochemical detection results

**Table 19. Antibody concentration and physicochemical detection results**

| Formulati on No. | Protein concentration | pH | Osmolality (mOsmol/kg) |
|---|---|---|---|
| | (mg/mL) | | |
| 4-1 | 184.83 | 5.80 | 349 |
| 4-2 | 185.54 | 5.80 | 331 |
| 4-3 | 180.12 | 5.80 | 366 |
| 4-4 | 175.34 | 5.84 | 316 |
| 4-5 | 181.57 | 5.83 | 359 |
| 4-6 | 179.59 | 5.84 | 379 |

### 2.3 light test

**Table 20. Detection results in samples under light (4500 ± 500 lx, 25 ± 2 °C)**

| Test item | | SEC-HPLC (%) | | | CE-SDS(NR) (%) | | | |
|---|---|---|---|---|---|---|---|---|
| Sampling time | Formulation No. | Aggregate | Monomer | Fragment | Fragment | HHL | Main peak | Aggregate |
| 0 d | 4-1 | 0.86 | 99.09 | 0.05 | 1.41 | 0.48 | 98.10 | 0.49 |
| | 4-2 | 0.82 | 99.14 | 0.04 | 1.42 | 0.43 | 98.11 | 0.47 |
| | 4-3 | 0.87 | 99.07 | 0.05 | 1.44 | 0.46 | 98.11 | 0.45 |
| | 4-4 | 0.93 | 98.99 | 0.08 | 1.47 | 0.45 | 98.14 | 0.39 |
| | 4-5 | 0.85 | 99.12 | 0.03 | 1.38 | 0.40 | 98.11 | 0.51 |
| | 4-6 | 0.91 | 99.05 | 0.04 | 1.35 | 0.46 | 98.17 | 0.48 |
| 7d | 4-1 | 2.93 | 96.97 | 0.10 | 3.16 | 1.70 | 95.59 | 1.25 |
| | 4-2 | 3.01 | 96.89 | 0.10 | 2.69 | 1.34 | 95.79 | 1.52 |
| | 4-3 | 2.75 | 97.17 | 0.08 | 2.23 | 1.10 | 96.38 | 1.39 |
| | 4-4 | 3.13 | 96.81 | 0.06 | 2.47 | 1.18 | 95.90 | 1.63 |
| | 4-5 | 2.66 | 97.27 | 0.07 | 2.21 | 1.04 | 96.39 | 1.40 |
| | 4-6 | 3.09 | 96.83 | 0.07 | 2.65 | 1.36 | 95.64 | 1.71 |
| 14 d | 4-1 | 5.33 | 94.49 | 0.18 | 5.29 | 2.82 | 92.45 | 2.26 |
| | 4-2 | 5.51 | 94.32 | 0.17 | 4.24 | 2.23 | 93.13 | 2.63 |
| | 4-3 | 6.10 | 93.75 | 0.15 | 3.94 | 2.19 | 93.06 | 3.00 |
| | 4-4 | 6.66 | 93.18 | 0.16 | 3.88 | 2.02 | 92.61 | 3.51 |
| | 4-5 | 5.85 | 93.98 | 0.17 | 4.22 | 2.07 | 92.74 | 3.04 |
| | 4-6 | 5.85 | 93.97 | 0.18 | 4.14 | 2.16 | 93.23 | 2.63 |

As can be seen from the data in Table 20, after 14 days of light, Formulation 4-4 exhibited a fast increase in SEC-HPLC aggregates, while the other formulation samples exhibited no significant differences in the SEC-HPLC detection results, indicating that samples with higher buffer concentrations have better stability. After 14 days of light, Formulation 4-1 exhibited a slightly faster increase in the CE-SDS (NR) fragments, while the other formulations exhibited no significant differences in the detection results.

**Table 21. Surfactant content assay results under light (4500 ± 500 lx, 25 ± 2 °C)**

| Formulation No. | Surfactant (µg/mL) | | Surfactant decrease rate after 14 d (%) |
|---|---|---|---|
| | 0 d | 14 d | |
| 4-1 | 2046 | 1478 | 28 |
| 4-2 | 331 | 394 | - |
| 4-3 | 2042 | 1749 | 14 |
| 4-4 | 2046 | 2032 | 1 |
| 4-5 | 2160 | 2030 | 6 |
| 4-6 | 1980 | 1687 | 15 |

As can be seen from the data in Table 21, proline and methionine exhibited better effects in inhibiting the degradation of PS80 than arginine hydrochloride under the light condition.

### 2.4 High-temperature test

**Table 22. Detection results in samples at high temperature (away from light, 40 ± 2 °C)**

| Test item | | SEC-HPLC (%) | | | IEC-HPLC (%) | | | CE-SDS(NR) (%) | | | |
|---|---|---|---|---|---|---|---|---|---|---|---|
| Sampling time | Formulation No. | Aggregate | Monomer | Fragment | Acidic variant | Main peak | Basic variant | Fragment | HHL | Main peak | Aggregate |
| 0 d | 4-1 | 0.86 | 99.09 | 0.05 | 21.63 | 73.02 | 5.35 | 1.41 | 0.48 | 98.10 | 0.49 |
| | 4-2 | 0.82 | 99.14 | 0.04 | 20.78 | 74.28 | 4.93 | 1.42 | 0.43 | 98.11 | 0.47 |
| | 4-3 | 0.87 | 99.07 | 0.05 | 22.00 | 73.30 | 4.70 | 1.44 | 0.46 | 98.11 | 0.45 |
| | 4-4 | 0.93 | 98.99 | 0.08 | 21.92 | 73.24 | 4.84 | 1.47 | 0.45 | 98.14 | 0.39 |
| | 4-5 | 0.85 | 99.12 | 0.03 | 21.54 | 73.76 | 4.70 | 1.38 | 0.40 | 98.11 | 0.51 |
| | 4-6 | 0.91 | 99.05 | 0.04 | 20.79 | 75.00 | 4.21 | 1.35 | 0.46 | 98.17 | 0.48 |
| 2W | 4-1 | 3.86 | 96.04 | 0.10 | 31.06 | 62.39 | 6.55 | 1.94 | 0.72 | 97.38 | 0.68 |
| | 4-2 | 1.24 | 98.69 | 0.07 | 27.45 | 66.74 | 5.82 | 1.87 | 0.56 | 97.64 | 0.49 |
| | 4-3 | 1.22 | 98.74 | 0.05 | 27.51 | 67.10 | 5.39 | 1.74 | 0.50 | 97.69 | 0.57 |
| | 4-4 | 1.24 | 98.72 | 0.04 | 28.16 | 66.45 | 5.39 | 1.88 | 0.66 | 97.55 | 0.57 |
| | 4-5 | 1.34 | 98.59 | 0.07 | 29.06 | 65.23 | 5.71 | 1.91 | 0.64 | 97.44 | 0.65 |
| | 4-6 | 1.24 | 98.70 | 0.07 | 27.74 | 66.20 | 6.06 | 1.84 | 0.59 | 97.55 | 0.61 |
| 4W | 4-1 | 11.12 | 88.62 | 0.26 | 42.77 | 47.89 | 9.34 | 3.82 | 1.37 | 94.81 | 1.37 |
| | 4-2 | 1.39 | 98.52 | 0.09 | 32.29 | 60.96 | 6.75 | 2.61 | 0.70 | 96.77 | 0.62 |
| | 4-3 | 1.29 | 98.66 | 0.05 | 33.45 | 60.08 | 6.47 | 2.48 | 0.62 | 96.91 | 0.61 |
| | 4-4 | 1.36 | 98.55 | 0.09 | 32.62 | 61.12 | 6.26 | 2.42 | 0.62 | 96.89 | 0.69 |
| | 4-5 | 1.45 | 98.38 | 0.17 | 34.99 | 59.59 | 5.42 | 2.61 | 0.66 | 96.76 | 0.63 |
| | 4-6 | 1.47 | 98.40 | 0.13 | 33.13 | 60.15 | 6.72 | 2.89 | 0.75 | 96.42 | 0.69 |

As can be seen from the data in Table 22: after 4 weeks of high-temperature test, Formulation 4-1 exhibited a significant decrease in monomer and a significant increase in aggregates by SEC-HPLC, and thus poor stability, while the other formulations exhibited no significant differences. After 4 weeks of high-temperature storage, Formulation 4-1 exhibited a significant decrease in the main peak and a significant increase in the acidic variants by IEC-HPLC and thus poor stability, while the other formulations exhibited no significant differences in the IEC-HPLC detection results. After 4 weeks of high-temperature storage, Formulation 4-1 exhibited a fast decrease in the main peak and a fast increase in the fragments by CE-SDS (NR) and thus poor stability, while the other formulations exhibited no significant differences.

**Table 23. Surfactant content assay results at high temperature (away from light, 40 ± 2 °C)**

| Formulation No. | Surfactant (µg/mL) | | | Surfactant decrease rate after 4 W (%) |
|---|---|---|---|---|
| | 0 d | 2W | 4W | |
| 4-1 | 2046 | 1432 | 1149 | 44 |
| 4-2 | 331 | 393 | 386 | - |
| 4-3 | 2042 | 1590 | 1131 | 45 |
| 4-4 | 2046 | 1725 | 1408 | 31 |
| 4-5 | 2160 | 1896 | 1516 | 30 |
| 4-6 | 1980 | 1198 | 928 | 53 |

As can be seen from the data in Table 23, proline and methionine also exhibited better effects in inhibiting the degradation of PS80 than arginine hydrochloride under the high-temperature condition.

### 2.5 Accelerated test

**Table 24. Detection results in samples under accelerated conditions (25 ± 2 °C)**

| Test item | | SEC-HPLC (%) | | | IEC-HPLC (%) | | | CE-SDS(NR) (%) | | | |
|---|---|---|---|---|---|---|---|---|---|---|---|
| Sampling time | Formulation No. | Aggregate | Monomer | Fragment | Acidic variant | Main peak | Basic variant | Fragment | HHL | Main peak | Aggregate |
| 0 d | 4-1 | 0.86 | 99.09 | 0.05 | 21.63 | 73.02 | 5.35 | 1.41 | 0.48 | 98.10 | 0.49 |
| | 4-2 | 0.82 | 99.14 | 0.04 | 20.78 | 74.28 | 4.93 | 1.42 | 0.43 | 98.11 | 0.47 |
| | 4-3 | 0.87 | 99.07 | 0.05 | 22.00 | 73.30 | 4.70 | 1.44 | 0.46 | 98.11 | 0.45 |
| | 4-4 | 0.93 | 98.99 | 0.08 | 21.92 | 73.24 | 4.84 | 1.47 | 0.45 | 98.14 | 0.39 |
| | 4-5 | 0.85 | 99.12 | 0.03 | 21.54 | 73.76 | 4.70 | 1.38 | 0.40 | 98.11 | 0.51 |
| | 4-6 | 0.91 | 99.05 | 0.04 | 20.79 | 75.00 | 4.21 | 1.35 | 0.46 | 98.17 | 0.48 |
| 1M | 4-1 | 0.73 | 99.26 | 0.01 | 28.63 | 65.37 | 5.99 | 2.06 | 0.71 | 97.46 | 0.48 |
| | 4-2 | 0.82 | 99.08 | 0.10 | 24.80 | 70.08 | 5.12 | 1.98 | 0.50 | 97.55 | 0.47 |
| | 4-3 | 0.82 | 99.14 | 0.04 | 24.82 | 70.12 | 5.06 | 1.87 | 0.52 | 97.66 | 0.47 |
| | 4-4 | 0.87 | 98.87 | 0.26 | 25.32 | 69.87 | 4.81 | 1.85 | 0.45 | 97.66 | 0.49 |
| | 4-5 | 0.86 | 99.05 | 0.09 | 25.09 | 69.49 | 5.42 | 1.87 | 0.43 | 97.66 | 0.47 |
| | 4-6 | 0.88 | 99.08 | 0.04 | 24.20 | 70.99 | 4.81 | 1.92 | 0.45 | 97.62 | 0.46 |
| 2M | 4-1 | 1.03 | 98.79 | 0.18 | 33.38 | 60.26 | 6.35 | 2.31 | 0.94 | 97.20 | 0.49 |
| | 4-2 | 1.06 | 98.84 | 0.11 | 28.65 | 65.74 | 5.60 | 1.62 | 0.46 | 97.88 | 0.50 |
| | 4-3 | 1.11 | 98.74 | 0.14 | 31.79 | 63.08 | 5.13 | 1.77 | 0.57 | 97.69 | 0.54 |
| | 4-4 | 1.18 | 98.69 | 0.13 | 28.71 | 65.64 | 5.65 | 1.63 | 0.44 | 97.82 | 0.55 |
| | 4-5 | 1.25 | 98.61 | 0.14 | 29.17 | 65.49 | 5.34 | 1.57 | 0.44 | 97.87 | 0.56 |
| | 4-6 | 1.15 | 98.72 | 0.13 | 27.83 | 66.68 | 5.49 | 1.65 | 0.46 | 97.79 | 0.56 |
| 3M | 4-1 | 1.05 | 98.75 | 0.20 | 34.54 | 58.59 | 6.87 | 2.58 | 1.03 | 96.87 | 0.55 |
| | 4-2 | 1.28 | 98.55 | 0.16 | 35.17 | 58.09 | 6.74 | 2.04 | 0.77 | 97.46 | 0.50 |
| | 4-3 | 1.07 | 98.80 | 0.12 | 33.86 | 60.78 | 5.36 | 2.12 | 0.80 | 97.36 | 0.52 |
| | 4-4 | 1.21 | 98.65 | 0.15 | 30.41 | 63.94 | 5.65 | 1.73 | 0.47 | 97.76 | 0.51 |
| | 4-5 | 1.22 | 98.64 | 0.14 | 30.74 | 63.85 | 5.42 | 1.70 | 0.50 | 97.78 | 0.52 |
| | 4-6 | 1.21 | 98.64 | 0.15 | 29.83 | 64.61 | 5.56 | 1.76 | 0.49 | 97.67 | 0.57 |

As can be seen from the data in Table 24, after 3 months of accelerated test, the SEC-HPLC monomer purities of the formulations were 98.5% or higher, suggesting good stability; the formulations exhibited no significant differences. After 3 months of acceleration, Formulations 4-4, 4-5, and 4-6 exhibited slower main peak decreases and acidic variant increases by IEC-HPLC. After 3 months of acceleration, Formulations 4-4, 4-5, and 4-6 exhibited slower main peak decreases and fragment increases by CE-SDS (NR).

**Table 25. Surfactant content assay results under accelerated conditions (25 ± 2 °C)**

| Formulation No. | Surfactant (µg/mL) | | Surfactant decrease rate after 3 M (%) |
|---|---|---|---|
| | 0 d | 3 M | |
| 4-1 | 2046 | 455 | 78 |
| 4-2 | 331 | 417 | - |
| 4-3 | 2042 | 1211 | 41 |
| 4-4 | 2046 | 1605 | 22 |
| 4-5 | 2160 | 1439 | 33 |
| 4-6 | 1980 | 1090 | 45 |

As can be seen from the data in Table 25, proline and methionine also exhibited better effects in inhibiting the degradation of PS80 than arginine hydrochloride under the accelerated condition. In summary, Formulation 4-1 had the lowest viscosity as compared to the other formulations, but was less stable under the various investigated conditions. Proline and methionine are advantageous for PS80 stabilization.

### Example 5: Formulation stability study

### 1. Protein concentration and excipient concentration test

The stability of formulations at two protein concentrations of 175 mg/mL and 150 mg/mL under conditions of different buffer concentrations and different proline concentrations was investigated, so as to explore the change ranges of viscosity and osmolality and confirm the stability of the formulations.

### 1.1 Procedures

The components and contents of the formulations are shown in Table 26. The experimental conditions and the detection items are shown in Table 27.

**Table 26. Formulation design**

| Formulation No. | Buffer | Stabilizer | Surfactant | Concentration of anti-IL-4Rα antibody (mg/mL) | pH |
|---|---|---|---|---|---|
| 5-1 | Histidine, 10 mM + acetic acid, 20 + 5 mM | Proline, 191 mM (2.2%) + methionine, 10 mM | | 175 | |
| 5-2 | | | 0.2% PS80 | 150 | 5.9±0.3 |
| 5-3 | Histidine, 20 mM + acetic acid, 26 ± 5 mM | Proline, 217 mM (2.5%) + methionine, 10 mM | | 175 | |
| 5-4 | | | | 150 | |

**Table 27. Experimental conditions and detection items**

| Experimental condition | Sampling time | Test item |
|---|---|---|
| light (4500 ± 500 lx, 25 ± 2 °C) | 0 d, 7 d, 14 d | PS80 content by SEC-HPLC, IEC-HPLC, CE-SDS (NR) |
| High temperature (away from light, 40 ± | 0 d, 2 W, 4 W | |

### 1.2 Results

### 1.2.1 Antibody concentration and physicochemical detection results

**Table 28. Antibody concentration and physicochemical detection results**

| Formulation No. | Protein concentration (mg/mL) | pH | Osmolality (mOsmol/kg) |
|---|---|---|---|
| 5-1 | 174.29 | 5.94 | 295 |
| 5-2 | 151.14 | 5.92 | 286 |
| 5-3 | 174.36 | 5.89 | 356 |
| 5-4 | 150.50 | 5.90 | 343 |

As can be seen from the data in Table 28, for formulation with the same excipients, the osmolality of the formulation with the protein concentration of 150 mg/mL was about 10 mOsmol/kg lower than that of the formulation with the protein concentration of 175 mg/mL; for formulations with the same protein concentration, when the buffer concentration was reduced by 10 mM and the proline concentration was reduced by 0.3%, the osmolality was reduced by about 60 mOsmol/kg. 1.2.2 Detection results of viscosity and sliding force

**Table 29. Detection results of viscosity and sliding force**

| Formulation No. | Viscosity (cP) | Syringe | Loading capacity | Initiation force (N) | Max continuous force (N) | Average continuous force (N) |
|---|---|---|---|---|---|---|
| 5-1 | 15.24 | 2.25 mL pre-filled syringe 1 (with a 27G1/2 syringe needle) | 1.14 mL | 12.84 | 15.04 | 13.88 |
| | | 2.25 mL pre-filled syringe 2 (with a 27G1/2 syringe needle) | | 11.10 | 12.66 | 12.10 |
| | | 1 mL elongated pre-filled syringe 1 (with a 27G1/2 syringe needle) | | 5.69 | 5.6 | 5.16 |
| 5-2 | 9.22 | 2.25 mL pre-filled syringe 1 (with a 27G1/2 syringe needle) | 2 mL | 8.63 | 10.04 | 9.28 |
| | | 2.25 mL pre-filled syringe 2 (with a 27G1/2 syringe needle) | | 7.95 | 9.90 | 9.26 |
| | | 1 mL elongated pre-filled syringe 1 (with a 27G1/2 syringe needle) | 0.67 mL | 4.30 | 4.14 | 3.81 |
| | | 1 mL elongated pre-filled syringe 2 (with a 27G1/2 syringe needle) | | 4.76 | 5.37 | 4.95 |
| 5-3 | 13.74 | 2.25 mL pre-filled syringe 1 (with a 27G1/2 syringe needle) | 1.14 mL | 9.90 | 10.96 | 10.27 |
| | | 2.25 mL pre-filled syringe 2 (with a 27G1/2 syringe needle) | | 10.91 | 11.93 | 11.49 |
| | | 1 mL elongated pre-filled syringe 1 (with a 27G1/2 syringe needle) | | 6.71 | 6.77 | 6.29 |
| 5-4 | 7.87 | 2.25 mL pre-filled syringe 1 (with a 27G1/2 syringe needle) | 2 mL | 7.13 | 8.57 | 7.79 |
| | | 2.25 mL pre-filled syringe 2 (with a 27G1/2 syringe needle) | | 7.56 | 8.80 | 8.31 |
| | | 1 mL elongated pre-filled syringe 1 (with a 27G1/2 syringe needle) | 0.67 mL | 4.95 | 4.88 | 4.64 |
| | | 1 mL elongated pre-filled syringe 2 (with a 27G1/2 syringe needle) | | 5.26 | 5.43 | 5.05 |

As can be seen from the data in Table 29, for formulations with the same protein concentration, when the buffer concentration was reduced by 10 mM and the proline concentration was reduced by 0.3%, the viscosity of the sample was increased by about 1.5 cP; for formulations with the same excipients, the viscosity of the formulation with the protein concentration of 150 mg/mL was about 6 cP lower than that of the formulation with 175 mg/mL; for the formulation with 175 mg/mL, the sliding force in the 1 mL elongate pre-filled syringe (with a 27G1/2 syringe needle) was about 50% lower than that in the 2.25 mL pre-filled syringe (with a 27G1/2 syringe needle). The viscosity and sliding force of the formulations in pre-filled syringes are within acceptable ranges.

### 1.2.3 light test

**Table 30. Sample content assay results under light (4500 ± 500 lx, 25 ± 2 °C)**

| Test item | | SEC-HPLC (%) | | | CE-SDS(NR) (%) | | | |
|---|---|---|---|---|---|---|---|---|
| Sampling time | Formulation No. | Aggregate | Monomer | Fragment | Fragment | HHL | Main peak | Aggregate |
| 0 d | 5-1 | 1.13 | 98.64 | 0.23 | 1.47 | 0.36 | 98.04 | 0.49 |
| | 5-2 | 1.10 | 98.70 | 0.20 | 1.46 | 0.41 | 98.05 | 0.49 |
| | 5-3 | 1.16 | 98.63 | 0.21 | 1.51 | 0.33 | 97.97 | 0.52 |
| | 5-4 | 1.15 | 98.64 | 0.21 | 1.51 | 0.40 | 97.96 | 0.53 |
| 7d | 5-1 | 4.34 | 95.34 | 0.31 | 2.85 | 1.07 | 95.01 | 2.14 |
| | 5-2 | 4.11 | 95.59 | 0.30 | 2.84 | 1.11 | 95.16 | 2.00 |
| | 5-3 | 3.47 | 96.23 | 0.30 | 2.99 | 1.15 | 95.30 | 1.71 |
| | 5-4 | 3.16 | 96.55 | 0.29 | 3.04 | 1.15 | 95.47 | 1.49 |
| 14 d | 5-1 | 6.99 | 92.66 | 0.35 | 3.59 | 1.45 | 92.57 | 3.84 |
| | 5-2 | 6.68 | 92.98 | 0.34 | 3.87 | 1.57 | 92.67 | 3.46 |
| | 5-3 | 5.95 | 93.69 | 0.36 | 4.05 | 1.58 | 93.08 | 2.87 |
| | 5-4 | 5.56 | 94.10 | 0.34 | 4.17 | 1.71 | 93.11 | 2.72 |

As can be seen from Table 30, after 14 days of light, Formulations 5-1 and 5-2 exhibited comparable SEC-HPLC monomer decreases, Formulations 5-3 and 5-4 exhibited comparable SEC-HPLC monomer decreases, and Formulations 5-3 and 5-4, i.e., formulations with high buffer concentrations and high proline contents, exhibited better stability. There were no significant differences in SEC-HPLC detection results between samples of the same formulation but different protein concentrations. After 14 days of light, the 4 formulations with different excipient concentrations and different protein concentrations exhibited basically consistent decreasing trends and amplitudes in the CE-SDS (NR) main peak, with no significant differences.

**Table 31. Polysorbate 80 content assay results under light (4500 ± 500 lx, 25 ± 2 °C)**

| Formulation No. | PS80 (µg/mL) | | | PS80 decrease rate after 14 d (%) |
|---|---|---|---|---|
| | 0 d | 7 d | 14 d | |
| 5-1 | 1960 | 1964 | 1851 | 6 |
| 5-2 | 1972 | 1958 | 1825 | 7 |
| 5-3 | 1944 | 1936 | 1851 | 5 |
| 5-4 | 1908 | 1918 | 1829 | 4 |

As can be seen from the data in Table 31, after 14 days of light, the PS80 content in the 4 formulations with different excipient concentrations and different protein concentrations was reduced by about 5%, suggesting good overall stability; there were no significant differences between the formulations.

### 1.2.4 High-temperature test

**Table 32. Detection results in samples at high temperature (away from light, 40 ± 2 °C)**

| Test item | | SEC-HPLC (%) | | | IEC-HPLC (%) | | | CE-SDS(NR) (%) | | | |
|---|---|---|---|---|---|---|---|---|---|---|---|
| Sampling time | Formulation No. | Aggregate | Monomer | Fragment | Acidic variant | Main peak | Basic variant | Fragment | HHL | Main peak | Aggregate |
| 0 d | 5-1 | 1.13 | 98.64 | 0.23 | 25.17 | 71.80 | 3.03 | 1.47 | 0.36 | 98.04 | 0.49 |
| | 5-2 | 1.10 | 98.70 | 0.20 | 25.96 | 71.12 | 2.93 | 1.46 | 0.41 | 98.05 | 0.49 |
| | 5-3 | 1.16 | 98.63 | 0.21 | 26.01 | 71.32 | 2.67 | 1.51 | 0.33 | 97.97 | 0.52 |
| | 5-4 | 1.15 | 98.64 | 0.21 | 25.12 | 71.86 | 3.02 | 1.51 | 0.40 | 97.96 | 0.53 |
| 2 W | 5-1 | 1.95 | 97.71 | 0.34 | 31.59 | 64.54 | 3.87 | 2.49 | 0.62 | 96.66 | 0.85 |
| | 5-2 | 1.86 | 97.79 | 0.35 | 31.96 | 63.96 | 4.08 | 2.51 | 0.59 | 96.87 | 0.62 |
| | 5-3 | 1.93 | 97.72 | 0.34 | 31.56 | 64.12 | 4.31 | 2.45 | 0.60 | 96.78 | 0.77 |
| | 5-4 | 1.86 | 97.80 | 0.34 | 31.81 | 64.10 | 4.09 | 2.52 | 0.60 | 96.81 | 0.67 |
| 4W | 5-1 | 2.19 | 97.48 | 0.33 | 39.65 | 55.84 | 4.51 | 2.51 | 0.60 | 96.62 | 0.87 |
| | 5-2 | 2.10 | 97.56 | 0.34 | 39.83 | 55.47 | 4.70 | 2.60 | 0.64 | 96.78 | 0.62 |
| | 5-3 | 2.18 | 97.49 | 0.33 | 40.15 | 55.04 | 4.81 | 2.58 | 0.66 | 96.67 | 0.75 |
| | 5-4 | 2.05 | 97.62 | 0.33 | 39.71 | 55.55 | 4.75 | 2.59 | 0.66 | 96.74 | 0.67 |

As can be seen from the data in Table 32: after 4 weeks of high-temperature storage, the 4 formulations with different excipient concentrations and different protein concentrations exhibited basically consistent decrease trends and amplitudes in purity by SEC-HPLC, IEC-HPLC, and CE-SDS (NR), and there were no significant differences, suggesting good overall stability.

**Table 33. Polysorbate 80 content assay results at high temperature (away from light, 40 ± 2 °C)**

| Formulation No. | PS80 (µg/mL) | | | PS80 decrease rate after 4 W (%) |
|---|---|---|---|---|
| | 0 d | 2 W | 4 W | |
| 5-1 | 1960 | 1695 | 1282 | 35 |
| 5-2 | 1972 | 1679 | 1371 | 30 |
| 5-3 | 1944 | 1646 | 1303 | 33 |
| 5-4 | 1908 | 1706 | 1305 | 32 |

As can be seen from the data in Table 33, after 4 weeks of high-temperature storage, the PS80 content in the 4 formulations with different excipient concentrations and different protein concentrations was reduced by about 30%; there were no significant differences between the formulations.

According to the analysis of the above results, the two protein concentrations of 150 mg/mL and 175 mg/mL exhibited good stability, with no significant differences. Formulations 5-3 and 5-4 exhibited slightly better stability than Formulations 5-1 and 5-2 under the light condition, but the differences were insignificant.

### 2. Accelerated and long-term stability test

### 2.1 Procedures

**Table 34. Experimental conditions and detection items**

| Experimental condition | Sampling time | Test item |
|---|---|---|
| Accelerated (away from light, 25 ± 2 °C) | 0 d, 1 M, 3 M, 6 M | SEC-HPLC, IEC-HPLC, CE-SDS(NR) |
| Long-term (away from light, 2-8 °C) | 6 M, 12 M | |

### 2.2 Results

### 2.2.1 Accelerated test

**Table 35. Detection results in samples under accelerated conditions (away from light, 25 ± 2 °C)**

| Test item | | SEC-HPLC (%) | | | IEC-HPLC (%) | | | CE-SDS(NR) (%) | | | |
|---|---|---|---|---|---|---|---|---|---|---|---|
| Sampling time | Formulation No. | Aggregate | Monomer | Fragment | Acidic variant | Main peak | Basic variant | Fragment | HHL | Main peak | Aggregate |
| 0 d | 5-4 | 1.50 | 98.47 | 0.03 | 24.47 | 71.42 | 4.12 | 1.53 | 0.46 | 97.89 | 0.58 |
| | 5-3 | 1.11 | 98.62 | 0.28 | 25.65 | 69.67 | 4.69 | 1.79 | 0.50 | 97.27 | 0.94 |
| 1 M | 5-4 | 1.26 | 98.53 | 0.21 | 24.56 | 69.85 | 5.59 | 1.89 | 0.51 | 97.39 | 0.72 |
| | 5-3 | 1.30 | 98.49 | 0.20 | 24.77 | 68.51 | 6.71 | 1.82 | 0.53 | 97.51 | 0.67 |
| 3 M | 5-4 | 1.47 | 98.24 | 0.29 | 30.71 | 65.15 | 4.15 | 2.14 | 0.51 | 97.24 | 0.62 |
| | 5-3 | 1.53 | 98.17 | 0.30 | 30.99 | 64.29 | 4.72 | 2.22 | 0.53 | 97.13 | 0.65 |
| 6 M | 5-4 | 1.66 | 98.00 | 0.34 | 38.45 | 57.09 | 4.46 | 2.69 | 0.80 | 96.58 | 0.73 |
| | 5-3 | 1.71 | 97.95 | 0.34 | 38.02 | 57.73 | 4.25 | 2.80 | 0.84 | 96.46 | 0.74 |

As can be seen from the data in Table 35, after the 6-month accelerated test at 25 °C, Formulations 5-4 and 5-3 both exhibited only a slight decrease in the SEC-HPLC monomer and CE-SDS (NR) main peak.

### 2.2.2 Long-term test

**Table 36. Detection results in samples under long-term conditions (away from light, 2-8 °C)**

| Test item | | SEC-HPLC (%) | | | IEC-HPLC (%) | | | CE-SDS(NR) (%) | | | |
|---|---|---|---|---|---|---|---|---|---|---|---|
| Sampling time | Formulation No. | Aggregate | Monomer | Fragment | Acidic variant | Main peak | Basic variant | Fragment | HHL | Main peak | Aggregate |
| 0 d | 5-4 | 1.50 | 98.47 | 0.03 | 24.47 | 71.42 | 4.12 | 1.53 | 0.46 | 97.89 | 0.58 |
| | 5-3 | 1.11 | 98.62 | 0.28 | 25.65 | 69.67 | 4.69 | 1.79 | 0.50 | 97.27 | 0.94 |
| 6 M | 5-4 | 1.27 | 98.43 | 0.30 | 24.90 | 71.86 | 3.25 | 1.67 | 0.54 | 97.85 | 0.48 |
| | 5-3 | 1.25 | 98.45 | 0.30 | 24.46 | 72.23 | 3.31 | 1.57 | 0.55 | 97.97 | 0.46 |
| 12 M | 5-4 | 1.35 | 98.40 | 0.26 | 26.86 | 69.15 | 3.99 | 1.56 | 0.46 | 97.95 | 0.49 |
| | 5-3 | 1.37 | 98.34 | 0.28 | 26.58 | 70.31 | 3.10 | 1.60 | 0.45 | 97.89 | 0.51 |

As can be seen from the data in Table 36, after the 12-month test at 2-8 °C, Formulations 5-4 and 5-3 exhibited no significant changes in the SEC-HPLC, IEC-HPLC, and CE-SDS (NR) purities, suggesting good long-term stability.

## Claims

1. An antibody formulation, comprising an anti-IL-4Rα antibody at 10-300 mg/mL or 10-200 mg/mL, a buffer, a stabilizer, and a surfactant, wherein the anti-IL-4Rα antibody comprises the heavy chain variable region set forth in SEQ ID NO: 1 and the light chain variable region set forth in SEQ ID NO: 2; the stabilizer is selected from proline, methionine, glycine, and a combination thereof; the pH of the antibody formulation is 5.0-7.0, 5.3-6.5, or 5.9 ± 0.3.

2. The antibody formulation according to claim 1, wherein the concentration of the stabilizer is 50-500 mM, 100-450 mM, 120-350 mM, or 145-330 mM.

3. The antibody formulation according to claim 1 or 2, wherein the stabilizer comprises proline at 100-300 mM; or, the stabilizer comprises proline at 180-230 mM; or, the stabilizer comprises proline at about 217 mM; or, the stabilizer comprises proline at about 191 mM.

4. An antibody formulation, comprising an anti-IL-4Rα antibody at 10-300 mg/mL or 10-200 mg/mL, a buffer, a stabilizer, and a surfactant, wherein the anti-IL-4Rα antibody comprises the heavy chain variable region set forth in SEQ ID NO: 1 and the light chain variable region set forth in SEQ ID NO: 2; the stabilizer comprises sucrose at 20-100 mM and arginine or a pharmaceutically acceptable salt thereof at 101-300 mM; the pH of the antibody formulation is 5.0-7.0, 5.3-6.5, or 5.9 ± 0.3.

5. The antibody formulation according to any one of claims 1-4, wherein the buffer is selected from histidine, citric acid, acetic acid, histidine-acetic acid, and histidine-citric acid.

6. The antibody formulation according to any one of claims 1-5, wherein the concentration of the buffer is 1-80 mM, 5-80 mM, 10-80 mM, 10-60 mM, 15-60 mM, or 20-55 mM.

7. The antibody formulation according to any one of claims 1-6, wherein the concentration of the anti-IL-4Rα antibody is 20-200 mg/mL, 40-200 mg/mL, 20-150 mg/mL, 100-200 mg/mL, 120-180 mg/mL, 145-180 mg/mL, or 20-80 mg/mL.

8. The antibody formulation according to any one of claims 2-7, further comprising methionine at 1-30 mM or EDTA at 0.01-0.5 mM.

9. The antibody formulation according to any one of claims 1-8, wherein the surfactant is polysorbate or poloxamer; or the surfactant is polysorbate 80, polysorbate 20, or poloxamer 188.

10. The antibody formulation according to any one of claims 1-9, wherein the concentration of the surfactant is 0.1-5 mg/mL, 0.5-5 mg/mL, or 0.5-3 mg/mL.

11. An antibody formulation, wherein the antibody formulation comprises an anti-IL-4Rα antibody at 20-250 mg/mL, a buffer at 5-80 mM, a stabilizer at 100-450 mM, and polysorbate 80 or poloxamer 188 at 0.1-5 mg/mL, and the pH of the antibody formulation is 5.3-6.5; or
the antibody formulation comprises an anti-IL-4Rα antibody at 20-200 mg/mL, a buffer at 5-80 mM, a stabilizer at 100-450 mM, and polysorbate 80 or poloxamer 188 at 0.1-5 mg/mL, and the pH of the antibody formulation is 5.3-6.5; or
the antibody formulation comprises an anti-IL-4Rα antibody at 20-100 mg/mL, a buffer at 10-30 mM, a stabilizer at 120-350 mM, and polysorbate 80 or poloxamer 188 at 0.5-3 mg/mL, and the pH of the antibody formulation is 5.3-6.5; or
the antibody formulation comprises an anti-IL-4Rα antibody at 45-55 mg/mL, a buffer at 10-30 mM, a stabilizer at 145-330 mM, and polysorbate 80 or poloxamer 188 at 1-3 mg/mL, and the pH of the antibody formulation is 5.3-6.5; or
the antibody formulation comprises an anti-IL-4Rα antibody at 100-200 mg/mL, a buffer at 5-80 mM, a stabilizer at 120-350 mM, and polysorbate 80 or poloxamer 188 at 0.5-3 mg/mL, and the pH of the antibody formulation is 5.3-6.5; or
the antibody formulation comprises an anti-IL-4Rα antibody at 130-200 mg/mL, a buffer at 10-60 mM, a stabilizer at 145-330 mM, and polysorbate 80 or poloxamer 188 at 0.5-3 mg/mL, and the pH of the antibody formulation is 5.3-6.5,
wherein the buffer is selected from histidine, citric acid, acetic acid, histidine-acetic acid, and histidine-citric acid;
the stabilizer is selected from proline, methionine, glycine, and a combination thereof;
the anti-IL-4Rα antibody comprises the heavy chain variable region set forth in SEQ ID NO: 1 and the light chain variable region set forth in SEQ ID NO: 2.

12. An antibody formulation, wherein the antibody formulation comprises an anti-IL-4Rα antibody at 20-250 mg/mL, a buffer at 5-80 mM, sucrose at 20-100 mM, arginine or a pharmaceutically acceptable salt thereof at 101-300 mM, and polysorbate 80 or poloxamer 188 at 0.1-5 mg/mL, and the pH of the antibody formulation is 5.3-6.5; or
the antibody formulation comprises an anti-IL-4Rα antibody at 20-200 mg/mL, a buffer at 5-80 mM, sucrose at 20-100 mM, arginine or a pharmaceutically acceptable salt thereof at 101-300 mM, and polysorbate 80 or poloxamer 188 at 0.1-5 mg/mL, and the pH of the antibody formulation is 5.3-6.5; or
the antibody formulation comprises an anti-IL-4Rα antibody at 20-200 mg/mL, a buffer at 5-80 mM, sucrose at 20-100 mM, arginine hydrochloride at 101-300 mM, and polysorbate 80 or poloxamer 188 at 0.5-3 mg/mL, and the pH of the antibody formulation is 5.3-6.5; or
the antibody formulation comprises an anti-IL-4Rα antibody at 45-55 mg/mL, a buffer at 10-30 mM, sucrose at 35-90 mM, arginine hydrochloride at 101-245 mM, and polysorbate 80 or poloxamer 188 at 1-3 mg/mL, and the pH of the antibody formulation is 5.3-6.5; or
the antibody formulation comprises an anti-IL-4Rα antibody at 100-200 mg/mL, a buffer at 5-80 mM, sucrose at 35-90 mM, arginine hydrochloride at 101-245 mM, and polysorbate 80 or poloxamer 188 at 0.5-3 mg/mL, and the pH of the antibody formulation is 5.3-6.5,
wherein the buffer is selected from histidine, citric acid, acetic acid, histidine-acetic acid, and histidine-citric acid;
the anti-IL-4Rα antibody comprises the heavy chain variable region set forth in SEQ ID NO: 1 and the light chain variable region set forth in SEQ ID NO: 2.

13. An antibody formulation, wherein
the antibody formulation comprises an anti-IL-4Rα antibody at about 150 mg/mL, histidine at about 20 mM, acetic acid at 26 ± 5 mM, proline at about 217 mM, methionine at about 10 mM, and polysorbate 80 at about 2 mg/mL, and the pH of the antibody formulation is 5.9 ± 0.3; or
the antibody formulation comprises an anti-IL-4Rα antibody at about 175 mg/mL, histidine at about 20 mM, acetic acid at 26 ± 5 mM, proline at about 217 mM, methionine at about 10 mM, and polysorbate 80 at about 2 mg/mL, and the pH of the antibody formulation is 5.9 ± 0.3; or
the antibody formulation comprises an anti-IL-4Rα antibody at about 150 mg/mL, histidine at about 10 mM, acetic acid at 20 ± 5 mM, proline at about 191 mM, methionine at about 10 mM, and polysorbate 80 at about 2 mg/mL, and the pH of the antibody formulation is 5.9 ± 0.3; or
the antibody formulation comprises an anti-IL-4Rα antibody at about 175 mg/mL, histidine at about 10 mM, acetic acid at 20 ± 5 mM, proline at about 191 mM, methionine at about 10 mM, and polysorbate 80 at about 2 mg/mL, and the pH of the antibody formulation is 5.9 ± 0.3; or
the antibody formulation comprises an anti-IL-4Rα antibody at about 175 mg/mL, histidine at about 20 mM, acetic acid at 33 ± 5 mM, proline at about 217 mM, methionine at about 10 mM, and polysorbate 80 at about 1 mg/mL, and the pH of the antibody formulation is 5.9 ± 0.3; or
the antibody formulation comprises an anti-IL-4Rα antibody at about 175 mg/mL, histidine at about 20 mM, arginine hydrochloride at about 110 mM, sucrose at about 44 mM, and polysorbate 80 at about 2 mg/mL, and the pH of the antibody formulation is 5.9 ± 0.3; or
the antibody formulation comprises an anti-IL-4Rα antibody at about 175 mg/mL, histidine at about 20 mM, arginine hydrochloride at about 110 mM, sucrose at about 44 mM, and poloxamer 188 at about 0.5 mg/mL, and the pH of the antibody formulation is 5.9 ± 0.3; or
the antibody formulation comprises an anti-IL-4Rα antibody at about 175 mg/mL, histidine at about 20 mM, proline at about 217 mM, methionine at about 10 mM, and polysorbate 80 at about 2 mg/mL, and the pH of the antibody formulation is 5.9 ± 0.3; or
the antibody formulation comprises an anti-IL-4Rα antibody at about 175 mg/mL, histidine at about 10 mM, proline at about 217 mM, methionine at about 10 mM, and polysorbate 80 at about 2 mg/mL, and the pH of the antibody formulation is 5.9 ± 0.3; or
the antibody formulation comprises an anti-IL-4Rα antibody at about 175 mg/mL, histidine at about 20 mM, acetic acid at 31 ± 5 mM, proline at about 217 mM, methionine at about 10 mM, and polysorbate 80 at about 2 mg/mL, and the pH of the antibody formulation is 5.9 ± 0.3; or
the antibody formulation comprises an anti-IL-4Rα antibody at about 50 mg/mL, histidine at about 20 mM, glycine at about 293 mM, and polysorbate 80 at about 2 mg/mL, and the pH of the antibody formulation is 5.9 ± 0.3; or
the antibody formulation comprises an anti-IL-4Rα antibody at about 50 mg/mL, histidine at about 20 mM, sucrose at about 88 mM, arginine hydrochloride at about 237 mM, and polysorbate 80 at about 2 mg/mL, and the pH of the antibody formulation is 5.9 ± 0.3; or
the antibody formulation comprises an anti-IL-4Rα antibody at about 50 mg/mL, histidine at about 20 mM, proline at about 217 mM, and polysorbate 80 at about 2 mg/mL, and the pH of the antibody formulation is 5.9 ± 0.3; or
the antibody formulation comprises an anti-IL-4Rα antibody at about 175 mg/mL, histidine at about 20 mM, arginine hydrochloride at about 110 mM, sucrose at about 44 mM, and polysorbate 80 at about 1 mg/mL, and the pH of the antibody formulation is 5.9 ± 0.3; or
the antibody formulation comprises an anti-IL-4Rα antibody at about 175 mg/mL, histidine at about 20 mM, arginine hydrochloride at about 110 mM, sucrose at about 44 mM, EDTA at about 0.05 mM, and polysorbate 80 at about 1 mg/mL, and the pH of the antibody formulation is 5.9 ± 0.3; or
the antibody formulation comprises an anti-IL-4Rα antibody at about 175 mg/mL, histidine at about 20 mM, arginine hydrochloride at about 110 mM, sucrose at about 44 mM, methionine at about 10 mM, and polysorbate 80 at about 1 mg/mL, and the pH of the antibody formulation is 5.9 ± 0.3; or
the antibody formulation comprises an anti-IL-4Rα antibody at about 175 mg/mL, histidine at about 20 mM, citric acid at about 20 mM, arginine hydrochloride at about 110 mM, sucrose at about 44 mM, and polysorbate 80 at about 1 mg/mL, and the pH of the antibody formulation is 5.9 ± 0.3,
wherein the anti-IL-4Rα antibody comprises the heavy chain variable region set forth in SEQ ID NO: 1 and the light chain variable region set forth in SEQ ID NO: 2.

14. The antibody formulation according to any one of claims 1-13, wherein the heavy chain of the anti-IL-4Rα antibody comprises the sequence set forth in SEQ ID NO: 3, or a sequence having at least 90% identity to the sequence set forth in SEQ ID NO: 3, or an amino acid sequence having one or more conservative amino acid substitutions as compared to the sequence set forth in SEQ ID NO: 3; and/or
the light chain of the anti-IL-4Rα antibody comprises the sequence set forth in SEQ ID NO: 4, or a sequence having at least 90% identity to the sequence set forth in SEQ ID NO: 4, or an amino acid sequence having one or more conservative amino acid substitutions as compared to the sequence set forth in SEQ ID NO: 4.

15. The antibody formulation according to any one of claims 1-14, wherein the anti-IL-4Rα antibody is dupilumab.

16. A method for preparing the antibody formulation according to any one of claims 1-15, comprising: taking components according to the formula, dissolving in water, mixing the mixture thoroughly, and adjusting the pH value to 5.0-7.0 to obtain the antibody formulation;
or, comprising: preparing a buffer, transferring the anti-IL-4Rα antibody into the buffer by ultrafiltration, adding an excipient, and diluting the antibody to a specified concentration to obtain the antibody formulation.

17. A lyophilized formulation, obtained by lyophilizing the antibody formulation according to any one of claims 1-15.

18. A reconstituted formulation, obtained by reconstituting the lyophilized formulation according to claim 17.

19. Use of the antibody formulation according to any one of claims 1-15, the lyophilized formulation according to claim 17, or the reconstituted formulation according to claim 18 in preparing a medicament for treating a disease, wherein the disease is a disease associated with IL-4R overexpression; or, the disease is an immune disease or disorder; or, the disease is selected from asthma, nasal polyp, eczema, sinusitis, chronic sinusitis, chronic sinusitis with nasal polyp, allergic dermatosis, chronic obstructive pulmonary disease, allergic rhinitis, arthritis, inflammatory disease, allergic reaction, autoimmune lymphoproliferative syndrome, autoimmune hemolytic anemia, Barrett's esophagus, autoimmune uveitis, tuberculosis, nephropathy, prurigo, prurigo nodularis, eosinophilic esophagitis, atopic dermatitis, urticaria, chronic urticaria, pruritus, bullous pemphigoid, allergic bronchopulmonary aspergillosis, neurodermitis, ulcerative colitis, milk anaphylaxis, and peanut allergy.

20. An article of manufacture, comprising the antibody formulation according to any one of claims 1-15, the lyophilized formulation according to claim 17, or the reconstituted formulation according to claim 18, and a container for storing the antibody formulation, the lyophilized formulation, or the reconstituted formulation.
